# EUROPEAN PATENT SPECIFICATION

(11) **EP 2 892 526 B1**
(45) Date of publication and mention of the grant of the patent: **29.05.2019**
(21) Application number: 13762088.6
(22) Date of filing: 06.09.2013
(51) Int. Cl.: A61K 31/343, A61K 31/353, A61K 31/352, A61K 36/484, A61P 1/02, A61P 17/00, A61K 8/49, A61K 8/97, A61Q 11/00, A61Q 15/00

(54) **COMPOSITION COMPRISING LICORICIDINE**
ZUBEREITUNG ENTHALTEND LICORIZIDIN
COMPOSITION CONTENANT DU LICORICIDINE

(30) Priority: 07.09.2012 EP 12183590; 07.09.2012 US 201261697884 P
(43) Date of publication of application: 15.07.2015
(73) Proprietor: BRAIN AG, 64673 Zwingenberg (DE)
(72) Inventor: KROHN, Michael, 64653 Lorsch (DE); MAMPEL, Jörg, 64625 Bensheim-Auerbach (DE); HAUSTEDT, Lars Ole, 14471 Potsdam (DE); KLUGE, Grit, 14959 Trebbin (DE)
(74) Representative: Herzog, Fiesser & Partner Patentanwälte PartG mbB
(86) International application number: PCT/EP2013/068512
(87) International publication number: WO 2014/037528

(56) References cited:
- EP-A1- 0 743 355
- US-A1- 2002 028 272
- US-A1- 2008 274 063
- US-A1- 2008 274 179
- KAJIYAMA K ET AL: "Flavonoids and isoflavonoids of chemotaxonomic significance from Glycyrrhiza pallidiflora (Leguminosae)", BIOCHEMICAL SYSTEMATICS AND ECOLOGY, PERGAMON PRESS, GB, vol. 21, no. 8, 1 December 1993 (1993-12-01), pages 785-793, XP025684120, ISSN: 0305-1978, DOI: 10.1016/0305-1978(93)90090-E [retrieved on 1993-12-01]
- TANAKA Y ET AL: "Antibacterial compounds of licorice against upper airway respiratory tract pathogens", JOURNAL OF NUTRITIONAL SCIENCE AND VITAMINOLOGY, UNIVERSITY OF TOKYO PRESS, TOKYO, JP, vol. 47, no. 3, 1 January 2001 (2001-01-01), pages 270-273, XP002970031, ISSN: 0301-4800
- TOSHIO FUKAI ET AL: "Antimicrobial activity of licorice flavonoids against methicillin-resistant Staphylococcus aureus", FITOTERAPIA, vol. 73, no. 6, 1 October 2002 (2002-10-01), pages 536-539, XP055041741, ISSN: 0367-326X, DOI: 10.1016/S0367-326X(02)00168-5
- TSUTOMU HATANO ET AL: "Phenolic constituents of licorice. VIII. Structures of glicophenone and glicoisoflavone and effects of licorice phenolics on methicillin-resistant staphylococcus aureus", CHEMICAL & PHARMACEUTICAL BULLETIN, PHARMACEUTICAL SOCIETY OF JAPAN, JP, vol. 48, no. 9, 1 January 2000 (2000-01-01), pages 1286-1292, XP002969544, ISSN: 0009-2363
- STEFAN GAFNER ET AL: "Isoflavonoids and Coumarins from Glycyrrhiza uralensis : Antibacterial Activity against Oral Pathogens and Conversion of Isoflavans into Isoflavan-Quinones during Purification", JOURNAL OF NATURAL PRODUCTS, vol. 74, no. 12, 27 December 2011 (2011-12-27), pages 2514-2519, XP055041719, ISSN: 0163-3864, DOI: 10.1021/np2004775

## Description

The present invention relates to a composition A comprising licoricidine and at least one component selected from the group consisting of glyasperin D, glyasperin C, gancaonin I and glycyrrhisoflavone, preferably wherein said composition A is a *Glycyrrhiza pallidiflora, Glycyrrhiza uralensis or Glycyrrhiza glabra* extract, and to a method for preparing the same. Furthermore the present invention relates to pharmaceutical or cosmetic composition or a method for preparing the same, said pharmaceutical or cosmetic composition comprising the composition A, wherein said composition A is preferably a *Glycyrrhiza pallidiflora, Glycyrrhiza uralensis or Glycyrrhiza glabra* extract. Furthermore, the present invention relates to a pharmaceutical or cosmetic composition, as described above, for body and oral care, in particular for use as deodorant or for use in treating or preventing dental caries.

It is known in the art that specific antibacterial agents may be useful for use as a deodorant or for preventing dental caries.

The human skin is populated by a multiplicity of different bacteria. The majority of these bacteria are not pathogenic and irrelevant for the odor of the skin. Others, on the other hand, are capable of decomposing secretions produced by the body, which can result in body odor The microorganisms which can cause body odor include, e.g, *Staphylococcus epidermidis, Corynebacterium xerosis, and Propionibacteria* and *Brevibacterium epidermidis (see e.g.* Martin et al., Journal of Investigative Dermatology (2010) 130, 529-540). One strategy to prevent or control the formation of unpleasant odor is to use compositions, such as deodorants, which comprise antimicrobial active compounds, as described above, which, because of their action, destroy, or at least decisively inhibit the reproduction of, the bacteria which lead to the formation of the unpleasant odour substances.

In a similar way, specific antibacterial agents were described to be useful for dental care since caries, plaques and further oral inflammations are believed to be caused by bacteria such as *Actinomyces viscosus, Streptococcus mutans, Fusobacterium nucleatum, and Porphyromonas gingivalis* in the mouth. For example, plaque, which is a soft deposit which forms on the surfaces of teeth, is comprised of an accumulation of bacteria and bacterial by-products. Further dental plaque is generally believed to be formed as a byproduct of bacterial growth and comprises a dense microbial layer consisting of a mass of microorganisms embedded in a polysaccharide matrix. Further, gingivitis is the inflammation or infection of the gums and the alveolar bones that support the teeth. Gingivitis is generally also believed to be caused by bacteria in the mouth (particularly the bacteria instigated in plaque formation) and the toxins formed as by products from the bacteria. In a similar way, periodontitis is generally believed to occur where unremoved plaque hardens into calculus (tartar) which affects the periodontal ligaments. Periodontitis is a progressively worsened state of disease as compared to gingivitis. As plaque and calculus continue to build up, the gums begin to recede from the teeth and pockets form there between, which ultimately may result in destruction of the bone and periodontal ligament. These reactions lead to the destruction of the supporting structure, continued infection, and potentially the subsequent loss of teeth.

Thus, specific antibacterial agents have been suggested in the art to retard plaque formation and the oral infections associated with plaque formation (see e.g. US 2006/0134024 A1).

A prominent example of an antimicrobial active compound against organisms forming body odor is the substance farnesol described in this context see DE 27 28 921 A1 and DE 33 15 058.

US 7,247,295 describes the use of 1,2-decanediol ins body care products for the control or prevention of body odor.

Further, antibacterial compounds derived from plant extracts are described in the art, such as in US 2006/0134024 A1 and US 2008/0274063 A1.

However, it is difficult to predict the efficacy of antibacterial compounds when incorporated into an oral care or body care composition with other active ingredients. Further, many antibacterial agents negatively interact with one or more components in these compositions and are not stable harmless from the dermatological standpoint.

Thus, notwithstanding the efficacy of certain antibacterial agents, there is a continuing interest in the oral care and body care field for compositions with advantageous antibacterial properties.

### SUMMARY OF THE INVENTION

The present invention relates to a composition A comprising licoricidine and at least one component selected from the group consisting of glyasperin D, glyasperin C, gancaonin I and glycyrrhisoflavone, a solvent wherein the solvent is a plant oil and butylhydroxyanisol.

In a preferred aspect, the present invention relates to a composition A comprising licoricidine and at least one component selected from the group consisting of glyasperin D, glyasperin C, gancaonin I and glycyrrhisoflavone, wherein said composition is a *Glycyrrhiza pallidiflora, Glycyrrhiza uralensis or Glycyrrhiza glabra* extract, preferably a *Glycyrrhiza pallidiflora* or *Glycyrrhiza uralensis* extract, more preferably a *Glycyrrhiza pallidiflora* extract.

Furthermore, the present invention relates to a method for the preparation of a *Glycyrrhiza pallidiflora, Glycyrrhiza uralensis or Glycyrrhiza glabra* extract, preferably a *Glycyrrhiza pallidiflora* or *Glycyrrhiza uralensis* extract, more preferably a *Glycyrrhiza pallidiflora* extract, comprising
(a) providing *Glycyrrhiza pallidiflora, Glycyrrhiza uralensis or Glycyrrhiza glabra,* preferably *Glycyrrhiza pallidiflora* or *Glycyrrhiza uralensis,* more preferably *Glycyrrhiza pallidiflora,* more preferably a branch thereof, and contacting the respective *Glycyrrhiza* with a liquid S1 thereby forming a liquid phase L1 and a solid residue R0;
(b) separating L1 from R0;
(c) optionally drying L1 to give a residue R1;.
(d) optionally dissolving the R1 in a liquid S2
to give the *Glycyrrhiza pallidiflora, Glycyrrhiza uralensis or Glycyrrhiza glabra* extract, preferably the *Glycyrrhiza pallidiflora* or *Glycyrrhiza uralensis* extract, more preferably the *Glycyrrhiza pallidiflora* extract.

In a further aspect, the present invention relates to a method for the preparation of a *Glycyrrhiza pallidiflora, Glycyrrhiza uralensis or Glycyrrhiza glabra* extract, preferably a *Glycyrrhiza pallidiflora* or *Glycyrrhiza uralensis* extract, more preferably a *Glycyrrhiza pallidiflora* extract,,
comprising
(a1) providing *Glycyrrhiza pallidiflora, Glycyrrhiza uralensis or Glycyrrhiza glabra,* preferably *Glycyrrhiza pallidiflora* or *Glycyrrhiza uralensis,* more preferably *Glycyrrhiza pallidiflora*, more preferably a branch thereof, and contacting the respective *Glycyrrhiza* with oil seeds;
(a2) subjecting the mixture according to (i) to co-pressing to give a liquid phase L1 and a solid residue R0,
(a3) separating L1 from R0
to give the *Glycyrrhiza pallidiflora, Glycyrrhiza uralensis or Glycyrrhiza glabra* extract, preferably the *Glycyrrhiza pallidiflora* or *Glycyrrhiza uralensis* extract, more preferably the *Glycyrrhiza pallidiflora* extract.

Furthermore, the present invention relates to a *Glycyrrhiza pallidiflora, Glycyrrhiza uralensis or Glycyrrhiza glabra* extract, preferably a *Glycyrrhiza pallidiflora* or *Glycyrrhiza uralensis* extract, more preferably a *Glycyrrhiza pallidiflora* extract, obtainable or obtained by a method, as described above.

The present invention furthermore relates to a pharmaceutical or cosmetic composition comprising the composition A described above and one or more cosmetically and/or pharmaceutically acceptable carriers and/or excipients.

The present invention furthermore relates to pharmaceutical or cosmetic composition comprising licoricidine and at least one component selected from the group consisting of glyasperin D, glyasperin C, gancaonin I and glycyrrhisoflavone, or comprising a *Glycyrrhiza pallidiflora, Glycyrrhiza uralensis or Glycyrrhiza glabra* extract, preferably a *Glycyrrhiza pallidiflora* or *Glycyrrhiza uralensis* extract, more preferably a *Glycyrrhiza pallidiflora* extract, as described above, and one or more cosmetically and/or pharmaceutically acceptable carriers and/or excipients for use in treating or preventing dental caries.

In a further aspect, the present invention relates to a method for the preparation of pharmaceutical or cosmetic composition, as described hereinabove, the method comprising mixing a composition A comprising licoricidine and at least one component selected from the group consisting of glyasperin D, glyasperin C, gancaonin I and glycyrrhisoflavone or a *Glycyrrhiza pallidiflora, Glycyrrhiza uralensis or Glycyrrhiza glabra* extract, preferably a *Glycyrrhiza pallidiflora* or *Glycyrrhiza uralensis* extract, more preferably a *Glycyrrhiza pallidiflora* extract, with one or more cosmetically and/or pharmaceutically acceptable carriers and/or excipients.

In a further aspect, the present invention relates to the use of a pharmaceutical or cosmetic composition for body and/or oral care, in particular in a method of treating or preventing dental caries. Thus, the present invention also relates to a pharmaceutical or cosmetic composition, as described above, for use in treating or preventing dental caries.

### DETAILED DESCRIPTION

Therefore, in a first aspect, the present invention relates to a composition A comprising licoricidine and at least one component selected from the group consisting of glyasperin D, glyasperin C, gancaonin I and glycyrrhisoflavone, a solvent wherein the solvent is a plant oil and butylhydroxyanisol.

It was surprisingly found that besides licoricidine, glyasperin D, glyasperin C, gancaonin I and glycyrrhisoflavone show antibacterial and/or anti-inflammatory and/or antifungal activity and thus improve the cosmetic and(or pharmaceutical benefits of a composition when used for oral or body care (see example 7).

In particular, it is contemplated that the composition of the invention may inhibit the growth of organisms, that is in particular bacteria, which cause body odor. Further, the compositions may be used for treatment or prevention of dental caries.

Preferably, the composition A comprises licoricidine and at least one, preferably at least two, more preferably at least three component(s) selected from the group consisting of glyasperin D, glyasperin C, gancaonin I and glycyrrhisoflavone.

According to a preferred embodiment, the composition does not comprise glycyrrhizic acid.

As to the amount of licoricidine present in the composition A of the invention, the composition A preferably comprises licoricidine in an amount of at least 0.001% by weight, more preferably in an amount in the range of from 0.005 to 50% by weight, more preferably in an amount in the range of from 0.01 to 30% by weigh and even more preferably in an amount in the range of from 0.05 to 10% by weight, based on the total weight of the composition.

In case the composition A comprises glyasperin D, A preferably comprises glyasperin D in an amount of at least 0.001% by weight, more preferably in an amount in the range of from 0.005 to 45% by weight, more preferably in an amount in the range of from 0.01 to 25% by weigh and even more preferably in an amount in the range of from 0.04 to 8% by weight, based on the total weight of the composition A.

In case the composition A comprises glyasperin C, the composition A preferably comprises glyasperin C in an amount of at least 0.001% by weight, more preferably in an amount in the range of from 0.0025 to 20% by weight, more preferably in an amount in the range of from 0.005 to 10% by weigh and even more preferably in an amount in the range of from 0.01 to 4% by weight, based on the total weight of the composition A.

In case the composition A comprises gancaonin I, the composition A preferably comprises gancaonin I in an amount of at least 0.001% by weight, more preferably in an amount in the range of from 0.005 to 40% by weight, more preferably in an amount in the range of from 0.01 to 20% by weigh and even more preferably in an amount in the range of from 0.025 to 5 % by weight, based on the total weight of the composition A.

In case the composition A comprises glycyrrhisoflavone, the composition A preferably comprises glycyrrhisoflavone in an amount of at least 0.001% by weight, more preferably in an amount in the range of from 0.0025 to 20% by weight, more preferably in an amount in the range of from 0.005 to 10% by weigh and even more preferably in an amount in the range of from 0.01 to 4% by weight, based on the total weight of the composition A.

According to a preferred aspect of the invention, the composition A comprises licoricidine, glyasperin D, glyasperin C, gancaonin I and glycyrrhisoflavone.

In this case, the composition A preferably comprises licoricidine in an amount the range of from 0.05 to 10% by weight, glyasperin D in an amount in the range of from 0.04 to 8% by weight, glyasperin C in an amount in the range of from 0.01 to 4% by weight, gancaonin I in an amount in the range of from 0.025 to 5% by weight and and glycyrrhisoflavone in an amount in the range of from 0.01 to 4% by weight, based on the total weight of the composition A.

According to a further preferred embodiment of the invention, the composition described above is a *Glycyrrhiza pallidiflora, Glycyrrhiza uralensis or Glycyrrhiza glabra* extract, preferably a *Glycyrrhiza pallidiflora* or *Glycyrrhiza uralensis* extract, more preferably a *Glycyrrhiza pallidiflora* extract, in particular an extract from *Glycyrrhiza pallidiflora Maxim.*

*Glycyrrhiza pallidiflora* is a papilionaceous herbaceous plant that belongs to the genus Glycyrrhiza (subfamily: Faboideae, family: Fabaceae). In the Taxonomy database, Glycyrrhiza pallidiflora has the Taxonomy ID: 74859. The Taxonomy database is, e.g., accessible via NCBI. Thus, the present invention also relates to a composition as described above, wherein the composition is a *Glycyrrhiza pallidiflora* extract. The extract is, for example, obtained or obtainable from the wooden parts and/or the root of *Glycyrrhiza pallidiflora,* more preferably from the wooden parts of *Glycyrrhiza pallidiflora.* Preferred wooden parts are those which are part of the central trunk of *Glycyrrhiza pallidiflora.* and parts such as branches and twigs. The terms "branches" and "twigs" are well known in the art. In botany, a branch is also referred to as "ramus", whereas a twig is a small branch which is also referred to as "ramulus". According to a preferred embodiment, the extract is obtained from branches and twigs.

*Glycyrrhiza uralensis* is also known as Chinese liquorice has the Taxonomy ID: 74613 and is e.g. commercially available from LGC Standards. Thus, the present invention also relates to a composition as described above, wherein the composition is a *Glycyrrhiza uralensis* extract. The extract is, for example, obtained or obtainable from the wooden parts and/or the root of *Glycyrrhiza uralensis,* more preferably from the roots of *Glycyrrhiza uralensis.*

*Glycyrrhiza glabra* has the Taxonomy ID: 48827. The root of *Glycyrrhiza glabra is known as liquorice or licorice.* Thus, the present invention also relates to a composition as described above, wherein the composition is a *Glycyrrhiza glabra* extract. The extract is, for example, obtained or obtainable from the wooden parts and/or the root of *Glycyrrhiza glabra*, more preferably from the roots of *Glycyrrhiza glabra.*

The term "*extract*" as used herein means a substance or composition obtained from the respective Glycyrrhiza which is obtained by extraction, maceration or percolation of the Glycyrrhiza material with a suitable solvent and, optionally, by partial or complete removal of the solvent or by co-pressing the Glycyrrhiza, preferably the *Glycyrrhiza pallidiflora*, with suitable oil seeds. Thus, extracts in accordance with this invention are either so-called co-pressed extracts or solvent-processed fluid extracts or so called dry *Glycyrrhiza* extracts obtained by evaporation of the whole liquid extract to dryness, e.g. by air drying, spray drying, vacuum oven drying, fluid-bed drying or freeze-drying, and optional washing and/or re-dissolving of this dry extract in at least one suitable solvent. Solvents suitable for extraction, percolation or maceration are known to those experienced in the art. Alkanes, alkanols, water, acetone and mixtures thereof as well as plant oils .are particularly suited. Carbon dioxide in fluid or super-critical form and pressurized gases with solvent properties are also suitable as extraction agents.

According to a preferred embodiment of the invention, no carbon dioxide in fluid or super-critical form and no pressurized gases with solvent properties are used as solvent for extraction, percolation or maceration. This has in particular advantages from the economical point of view.

According to a preferred embodiment of the invention, the *Glycyrrhiza* extract, preferably the *Glycyrrhiza pallidiflora,* extract comprises a solvent S1, wherein said solvent is the solvent used for extraction, maceration or percolation of the *Glycyrrhiza* material. According to an alternative embodiment of the invention, the *Glycyrrhiza* extract, preferably the *Glycyrrhiza pallidiflora* extract, comprises a solvent S2, wherein S2 is the solvent used for re-dissolving the dry extract.

Solvent S1 is a plant oil, preferably an edible plant oil, more preferably a colorless edible plant oil. In case S1 is a plant oil, the plant oil is preferably selected from the group consisting of safflower oil, sunflower oil, olive oil rapeseed oil and mixtures thereof. In case S1 is a plant oil, the method preferably does not comprise steps (c) and (d), more preferably consist of steps (a) and (b).

Solvent S2 is plant oil, more preferably a colorless edible plant oil. In case S2 is a plant oil, the plant oil is preferably selected from the group consisting of safflower oil, sunflower oil, olive oil rapeseed oil and mixtures thereof.

Preferably the *Glycyrrhiza* extract (i.e. *the Glycyrrhiza pallidiflora, Glycyrrhiza uralensis or Glycyrrhiza glabra* extract), preferably the *Glycyrrhiza pallidiflora* or *Glycyrrhiza uralensis* extract, more preferably the *Glycyrrhiza pallidiflora* extract, is obtained or obtainable by a process comprising
(a) providing *Glycyrrhiza pallidiflora, Glycyrrhiza uralensis or Glycyrrhiza glabra,* preferably *Glycyrrhiza pallidiflora* or *Glycyrrhiza uralensis,* more preferably *Glycyrrhiza pallidiflora,* more preferably a branch thereof, and contacting the *Glycyrrhiza material* with a liquid S1 thereby forming a liquid phase L1 and a solid residue R0;
(b) separating L1 from R0;
(c) optionally drying L1 to give a residue R1,
(d) optionally dissolving the R1 in a liquid S2,
to give the *Glycyrrhiza* extract, preferably the *Glycyrrhiza pallidiflora* or *Glycyrrhiza uralensis* extract, more preferably the *Glycyrrhiza pallidiflora* extract.

Thus, the present invention also relates to a method for preparing a *Glycyrrhiza* extract (i.e. *the Glycyrrhiza pallidiflora, Glycyrrhiza uralensis or Glycyrrhiza glabra* extract), preferably the *Glycyrrhiza pallidiflora* or *Glycyrrhiza uralensis* extract, more preferably the *Glycyrrhiza pallidiflora* extract, comprising
(a) providing *Glycyrrhiza pallidiflora, Glycyrrhiza uralensis or Glycyrrhiza glabra,* preferably *Glycyrrhiza pallidiflora* or *Glycyrrhiza uralensis,* more preferably *Glycyrrhiza pallidiflora,* more preferably a branch and/or a root thereof, and contacting the *Glycyrrhiza material* with a liquid S1 thereby forming a liquid phase L1 and a solid residue R0;
(b) separating L1 from R0;
(c) optionally drying L1 to give a residue R1,
(d) optionally dissolving the R1 in a liquid S2,
to give the *Glycyrrhiza* extract, preferably the *Glycyrrhiza pallidiflora* or *Glycyrrhiza uralensis* extract, more preferably the *Glycyrrhiza pallidiflora* extract.

### Step (a)

### Extraction, maceration or percolation

The contacting in step (a) is preferably carried out by extraction, maceration or percolation, more preferably the *Glycyrrhiza material* (i.e. *the Glycyrrhiza pallidiflora, Glycyrrhiza uralensis or Glycyrrhiza glabra* material), preferably *the Glycyrrhiza pallidiflora* is extracted with the liquid S1 or the contacting in (a) is carried out by a maceration process. The choice of the respective method usually depends on the nature of liquid S1 which is employed in step (a).

### Maceration:

According to one preferred embodiment of the invention, the contacting in step (a) is carried out by maceration. Thus, the present invention also relates to a method, as described above, and a *Glycyrrhiza* extract, preferably a *Glycyrrhiza pallidiflora extract*, obtained or obtainable by said method, as described above, wherein the contacting in (a) is carried out by a maceration.

In this case, the liquid S1 comprises a plant oil. The term "plant oil" refers to lipids obtained from plant sources.

Preferably the plant oil is an edible oil, more preferably a colorless edible oil.

Preferably, the plant oil is selected from the group consisting of safflower oil, sunflower oil, olive oil rapeseed oil and mixtures thereof.

More preferably S1 consists of a plant oil, preferably a edible plant oil, more preferably wherein said oil is colorless, most preferably wherein said oil is selected from the group consisting of safflower oil, sunflower oil, olive oil, rapeseed oil and mixtures thereof, more preferably from the group consisting of safflower oil, sunflower oil, rapeseed oil and mixtures thereof.

In case the contacting is carried out by maceration, S1 and the *Glycyrrhiza* material, preferably the *Glycyrrhiza pallidiflora* material, is preferably allowed to stand for a time in the range of from 1 to 120 hours, preferably in the range of from 1 day to 4 days, more preferably about 24 to 72 hours, in particular at a temperature in the range of from 10 to 60 °C, more preferably at a temperature in the range of from 20 to 50 °C, most preferably at room temperature.

In case step (a) is carried out by maceration using a plant oil as solvent S1, the method preferably does not comprise steps (c) and (d), more preferably the method consist of steps (a) and (b).

### Extraction:

According to a further preferred embodiment, the contacting in step (a) is carried out by extraction. Thus, according to one preferred embodiment, the present invention also relates to a method, as described above, and a *Glycyrrhiza* extract, preferably a *Glycyrrhiza pallidiflora or Glycyrrhiza uralensis* extract, more preferably a *Glycyrrhiza pallidiflora* extract obtained or obtainable by said method, as described above, wherein in (a) *Glycyrrhiza pallidiflora, Glycyrrhiza uralensis or Glycyrrhiza glabra,* preferably *Glycyrrhiza pallidiflora,* is extracted with the liquid S1.

There are no particular restriction as to the extraction procedure, thus, any extraction method known to those skilled in the art, such as ultrasonic assisted extraction, soxhlet extraction, microwave assisted extraction and the like, may be used. Preferably the extraction is carried out at a temperature in the range of from 0°C to 100 °C, preferably in the range of from 10 °C to 50 °C, more preferably at room temperature.

Preferably, the ratio of amount of *Glycyrrhiza pallidiflora, Glycyrrhiza uralensis or Glycyrrhiza glabra,* preferably *Glycyrrhiza pallidiflora,* (weight) to solvent S1 (weight) is in the range of from 1:2 to 1:20, more preferably in the range of about 1:5.

The extraction can be carried out in one or more extraction steps. Preferably a multi-stage extraction is carried out in which a multiplicity of separating stages connected in series is used.

As described above, in step (a) a liquid phase L1 comprising the active ingredients, in particular comprising licoricidine and at least one component selected from the group consisting of glyasperin D, glyasperin C, gancaonin I and glycyrrhisoflavone, more licoricidine, glyasperin D, glyasperin C, gancaonin I and glycyrrhisoflavone, and the solvent S1 is formed. Further a solid residue R0 is obtained, said solid residue being the remaining solid material of *Glycyrrhiza pallidiflora*

### Step b)

After the extraction, described above, the liquid phase L1 is separated from the solid residue R0.

The separation step may be carried out by any suitable method known to those skilled in the art. According to one embodiment of the invention, the separation is carried out by filtration. The term "filtration" or "filtering" refers to the process of removing essentially all, preferably all, of the solid residue R0, which may be present as suspended particles, from the liquid phase by passing the composition through one or more membranes or filters. According to a preferred embodiment, the resulting liquid phase L1 or an optionally concentrated and/or further purified L1 corresponds to the *Glycyrrhiza extract.* In this case S1 preferably comprises, in particular consist of a plant oil.

*Thus, according to this preferred embodiment, the* present invention relates to a method for preparing a *Glycyrrhiza* extract, and an extract obtained or obtainable by said method, as described above, the method comprising
(a) providing the *Glycyrrhiza* material, more preferably a branch and/or a root of *Glycyrrhiza pallidiflora* , and contacting the *Glycyrrhiza* material with a liquid S1 thereby forming a liquid phase L1 and a solid residue R0;
(b) separating L1 from R0,
g) optionally concentrating and/or purifying L1
to give the respective *Glycyrrhiza* extract (L1)

Thus, in this case, the method preferably does not comprise steps (c) and (d), more preferably the method consist of steps (a) and (b).

As described above, in step (g), L1 may be subjected to one or more further purification or work-up steps such as concentration of the extract and/or purifying the extract, e.g. filtering the extract to remove any undissolved material, to finally give the *Glycyrrhiza* extract.

According an alternative embodiment of the invention, the method comprises the steps (and does not comprise step (g)):
(c) drying L1 to give a residue R1;
(d) dissolving the R1 in a liquid S2.

Optional step c)

According to a further embodiment of the invention, L1 may, optional after further purification steps, be concentrated, in particular evaporated to dryness as mentioned above, thus e.g. by air drying, spray drying, vacuum oven drying, fluid-bed drying or freeze-drying to give residue R1. In the case in which L1 is evaporated to dryness, the residue R1 corresponds to the dry *Glycyrrhiza* extract, preferably the dry *Glycyrrhiza pallidiflora* extract mentioned above

Subsequent to the evaporating step, the method may comprise further steps, such as, e.g. at least one purification step and/or at least one homogenization step.

Thus, the present invention also relates to a method for preparing a *Glycyrrhiza pallidiflora, Glycyrrhiza uralensis or Glycyrrhiza glabra extract*, preferably a *Glycyrrhiza pallidiflora* or *Glycyrrhiza uralensis* extract, more preferably a Glycyrrhiza pallidiflora extract comprising
(a) providing *Glycyrrhiza pallidiflora, Glycyrrhiza uralensis or Glycyrrhiza glabra,* preferably *Glycyrrhiza pallidiflora* or *Glycyrrhiza uralensis,* more preferably *Glycyrrhiza pallidiflora,* more preferably a branch and/or a root thereof, and contacting the *Glycyrrhiza* material with a liquid S1 thereby forming a liquid phase L1 and a solid residue R0;
(b) separating L1 from R0;
(c) drying L1 to give a residue R1
to give the *Glycyrrhiza* extract, preferably the *Glycyrrhiza pallidiflora* extract.

### Optional step d)

As described above, according to one embodiment of the invention, residue R1, is preferably re-dissolved in a liquid S2, with S2 comprising a plant oil, the plant oil is preferably selected from the group consisting of safflower oil, sunflower oil, olive oil, rapeseed oil and mixtures thereof, more preferably from the group consisting of safflower oil, sunflower oil, rapeseed oil and mixtures thereof.

It was surprisingly found that licoricidine and the at least one component selected from the group consisting of glyasperin D, glyasperin C, gancaonin I and glycyrrhisoflavone are particularly stable in the obtained extract when using a plant oil as solvent S1. The term "stable" in this context means that essentially all of licoricidine and the other components selected from the group consisting of glyasperin D, glyasperin C, gancaonin I and glycyrrhisoflavone present within the composition are stable and e.g. not oxidized, preferably for a time of at least 4 weeks, more preferably of at least 8 weeks, and most preferably of at least 12 weeks.

Without being bound to any theory, it is assumed that the presence of unsaturated fatty acids in S1 may avoid or diminish any oxidative stress which often occurs when using usual methods known in the art and which may adversely affect the active ingredients in the *Glycyrrhiza.*

Thus, the present invention also relates to a method for preparing a *Glycyrrhiza* extract, preferably a *Glycyrrhiza pallidiflora* or *Glycyrrhiza uralensis* extract, more preferably a *Glycyrrhiza pallidiflora* extract,
comprising
(a) providing *Glycyrrhiza pallidiflora, Glycyrrhiza uralensis or Glycyrrhiza glabra,* preferably *Glycyrrhiza pallidiflora* or *Glycyrrhiza uralensis,* more preferably *Glycyrrhiza pallidiflora,* more preferably a branch and/or a root thereof, and contacting the *Glycyrrhiza* material with a liquid S1 thereby forming a liquid phase L1 and a solid residue R0;
(b) separating L1 from R0;
(c) drying L1 to give a residue R1,
(d) dissolving the R1 in a liquid S2,
to give the *Glycyrrhiza* extract, preferably the *Glycyrrhiza pallidiflora* extract.

Preferably, the ratio of amount of R1 (weight) to solvent S2 (weight) is in the range of from 1 : 5 to 1 : 50, more preferably in the range of about 1 : 10

Subsequent to the dissolving step d), the method may comprise further steps, such as, e.g. at least one purification step, such as filtering.

### Co-Pressing

According to a further preferred embodiment, the present invention relates to a method and to a composition A obtained or obtainable by said method, said method comprising
(a1) providing *Glycyrrhiza pallidiflora, Glycyrrhiza uralensis or Glycyrrhiza glabra,* preferably *Glycyrrhiza pallidiflora* or *Glycyrrhiza uralensis* extract, more preferably *Glycyrrhiza pallidiflora* more preferably a branch and/or a root thereof, and contacting the *Glycyrrhiza* material with oil seeds;
(a2) subjecting the mixture according to (i) to co-pressing to give a liquid phase L1 and a solid residue R0,
(a3) separating L1 from R0
to give the *Glycyrrhiza* extract, preferably the *Glycyrrhiza pallidiflora* extract.

It has been surprisingly found that a particularly stable composition A comprising a high concentration of active ingredients, i.e. licoricidine and the at least on component selected from the group consisting of glyasperin D, glyasperin C, gancaonin I and glycyrrhisoflavone, and a low concentration of fatty acids derived from *Glycyrrhiza pallidiflora* is obtained when using the above mentioned method comprising the steps (a1) to (a3). The term "stable" in this context means that essentially all of licoricidine and of the other component selected from the group consisting of glyasperin D, glyasperin C, gancaonin I and glycyrrhisoflavone present within the composition are stable, preferably for a time of at preferably for a time of at least 4 weeks, more preferably of at least 8 weeks, and most preferably of at least 12 weeks.

Again without being bound to any theory, it is assumed that the presence of unsaturated fatty acids in S1 may avoid or diminish any oxidative stress which often occurs when using usual methods known in the art and which may adversely affect the active ingredients in the *Glycyrrhiza.*

Further it is contemplated that the *Glycyrrhiza* extract produced in this manner is distinguished from *Glycyrrhiza* extract comprising conventionally produced scented or flavored oils by the fact that the fatty acid spectrum of the oilseeds used and their inherent vitamins are preserved.

Further, the amount of dissolved ingredients, i.e. of licoricidine and the at least on component selected from the group consisting of glyasperin D, glyasperin C, gancaonin I and glycyrrhisoflavone is high in the vegetable oils produced. When applying this process, L1 preferably comprises more then 20 weight %, more preferably more than 30 weight%, of all licoricidine which was present in the *Glycyrrhiza pallidiflora* material. Further the process is mild and there is no need to use any organic solvents. Thus, only edible compounds (oil and plant material) may be used, thus yielding in a non-toxic product.

As described above, in step (a2) a liquid phase L1 is formed, comprising the active components extracted from *Glycyrrhiza* and a plant oil derived from the respective oils seeds. It has to be understood that L1 may comprise various further substances derived from the respective *Glycyrrhiza.*

Further a solid residue R0 is obtained, said solid residue being the remaining solid material of *Glycyrrhiza* and of the employed oil seeds. According to a preferred embodiment, the oils seeds are selected from the group consisting of kernel seed, rape seed, sesame seed, sunflower seed and mixtures thereof. In particular, sunflower seeds are used.

Thus, the present invention also relates to a method and to a composition A obtained or obtainable by said method, said method comprising
(a1) providing *Glycyrrhiza pallidiflora, Glycyrrhiza uralensis or Glycyrrhiza glabra,* preferably *Glycyrrhiza pallidiflora* or *Glycyrrhiza uralensis,* more preferably *Glycyrrhiza pallidiflora,* and contacting the *Glycyrrhiza* material with sunflower seeds;
(a2) subjecting the mixture according to (i) to co-pressing to give a liquid phase L1 and a solid residue R0,
(a3) separating L1 from R0
to give the *Glycyrrhiza* extract, preferably the *Glycyrrhiza pallidiflora* extract.

In step (a1), the *Glycyrrhiza* material is preferably mixed with the oil seeds. The weight ratio of *Glycyrrhiza* material to oils seed is preferably in the range of from 99.9 : 0.1 to 50 : 50. more preferably in the range of from 1:2 to 1:3.

The Glycyrrhiza material, preferably the Glycyrrhiza pallidiflora material, is preferably provided in pieces having a maximum diameter of about 30 nm, such as in the range of from 1 nm to 20 nm. The provision of Glycyrrhiza material, preferably the Glycyrrhiza pallidiflora material in step (a1) thus may comprise a step of chopping or shredding the Glycyrrhiza material into pieces having a size in the range mentioned above.

It is to be understood that in this step, further components may be added such as further plant material, e.g. chopped or shredded parts of fragrant or aromatic plants such as, for instance, roses, lavender, violets, jasmine, vanilla, iris root, camomile (flores chamomilla) and others may be used for the production of scented or fragrant enriched vegetable oils.

Furthermore, as additional component, further plant material comprising antimicrobial active ingredients to be extracted from this material when using the above mentioned method, may be used in this step.

As mentioned above, the oil seed and the Glycyrrhiza material, preferably the Glycyrrhiza pallidiflora material, and optionally the additional components are co pressed in step (a2), preferably at a suitable temperature with a suitable pressure. A suitable co-pressing procedure is e.g. described in US 2002/0028272.

Preferably, the Glycyrrhiza material, more preferably the Glycyrrhiza pallidiflora material, is only compressed to a limited degree. The level of pressure exerted is such that while it does lead to breaking open or rupturing the plant cells it does not destroy them.

The temperature during step (a2) is kept at a level which ensures that no thermal damage of the components of the composition, in particular of licoricidine an the at least on component selected from the group consisting of glyasperin D, glyasperin C, gancaonin I and glycyrrhisoflavone occurs. Preferably the temperature is in the range of from 10 to 70 °C, more preferably in the range of from 15 to 50 °C, even more preferably in the range of from 20 to 30 °C. It is to be understood that during this step, the temperature may be varied or held essentially constant. Preferably the temperature is held essentially constant.

Step (a2) is preferably carried out in an oil press, for instance, such that its enthalpy, i.e. temperature as a result of the exerted pressure, does not exceed 60° C. The flow conditions generated in the cylinder of the oil press cause the oil extracted from the oilseeds to be repressed at the nozzle aperture of the oil press thus washing the oil soluble ingredients from the plant material. Advantageously, the compression cylinder of the oil press is encased by a container for receiving the oil and protecting it from detrimental ambient effects such as the oxygen in the air and especially for preventing escape of the highly volatile ingredients or essential oils.

US 2002/028272 mentions extracts of lipophilic compounds (essential oil compounds) obtained by co-pressing of essential oil containing plant parts with oil seeds (e.g. seeds from sunflower, rape or sesame).

EP0 743 355 discloses a method for obtaining oil mixtures comprising wheat germ oil characterized in that the wheat germs are mixed with at least one other species of oil-containing germ or seed before pressing.

### Pharmaceutical Composition:

As described above, the present invention further relates to a pharmaceutical or cosmetic composition comprising the composition A, as described above or a *Glycyrrhiza pallidiflora, Glycyrrhiza uralensis or Glycyrrhiza glabra extract*, preferably a *Glycyrrhiza pallidiflora* or *Glycyrrhiza uralensis* extract, more *preferably a Glycyrrhiza pallidiflora* extract, obtained or obtainable as described above, and additionally or more cosmetically and/or pharmaceutically acceptable carriers and/or excipients.

Preferably the amount of the composition A or the *Glycyrrhiza* extract obtained or obtainable as described above present in the final pharmaceutical or cosmetic composition is in the range of from 0.1 to 25 weight %, based on the total weight of the pharmaceutical or cosmetic composition.

It is to be understood that the preparation of the pharmaceutical or cosmetic composition of the invention preferably takes place under GMP standardized conditions in order to ensure quality, pharmaceutical security, and effectiveness of the pharmaceutical or cosmetic composition. Further criteria for an ingredient being pharmaceutically or cosmetically acceptable can be derived from approval regulations by a regulatory agency or other generally recognized pharmacopoeias.

According to an embodiment of the present invention, the pharmaceutical or cosmetic composition according to the invention is prepared by mixing the composition described above, or the *Glycyrrhiza* extract described above with one or more cosmetically and/or pharmaceutically acceptable carriers and/or excipients.

The pharmaceutical or cosmetic composition can in principle be prepared by combining all ingredients at suitable conditions known to those skilled in the art using any method known to those skilled in the art. In principle, any suitable order of adding the ingredients may be used. Every mixture obtained during the preparation process may be e.g. stirred and/or homogenized. In case a homogenization is carried out, this homogenization is carried out with a thorax mixer.

The choice of the suitable cosmetically and/or pharmaceutically acceptable carriers and/or excipients depends on the intended use of the composition. In general, the compositions of the invention may be formulated and provided in any suitable form which is advantageous and effective for consumer use.

The term "carrier or excipient" as used herein, means any suitable vehicle, which can be used to apply the present compositions to the skin or to use the composition as dental care in a safe and effective manner. A carrier may also reduce any undesirable side effects of the active compounds present in the composition. A suitable carrier is stable, i.e. e.g., incapable of reacting with other ingredients in the composition. The excipient and/or carrier must be "cosmetically and/or pharmaceutically acceptable" and "safe and effective" in the sense of being compatible with the other ingredients of the composition and not deleterious to the recipient thereof.

Examples of cosmetically and/or pharmaceutically acceptable excipients or carriers include stabilizers, further antibacterial agents, enzymes for reducing malodor, thickeners, lubricants, waxes, Vaseline, chelating agents, film formers, surfactants, diluents, antioxidants, binders, preservatives, coloring agents (such as pigments or dyes), moisture absorbents fragrances or emulsifiers or other conventional constituents of a cosmetic formulation, such as preferably alcohols, polyols, polymers, foam stabilizers, electrolytes, organic solvents or silicone derivatives. Cosmetically and/or pharmaceutically excipients may also include skin permeation enhancers.

### Body Care:

According to one preferred embodiment of the invention the cosmetically and/or pharmaceutically composition is used for body care. In this case, the cosmetically and/or pharmaceutically composition is preferably formulated for topical administration, in particular formulated as solution, emulsion, suspension, or dispersion in suitable pharmaceutical or cosmetical bases or carriers, according to conventional methods known in the art for preparation of various dosage forms. According to a preferred embodiment, the composition is a cream, an ointment, a gel or an emulsion.

Most preferably, the cosmetic and/or pharmaceutically composition is used for controlling body odor. Thus, the composition is preferably provided as deodorant. It has been surprisingly found that the composition A according to the invention and the cosmetic and/or pharmaceutically composition comprising said composition A provides good effectiveness against the microorganisms responsible for body odor (including underarm odor).

In particular, the composition according to the invention is effective against *Corynebacterium xerosis.*

Thus, the present invention also describes a method for inhibiting the growth of organisms causing body odor on a human or animal body, the method comprising the topical application of a composition as described above or of a pharmaceutical or cosmetic composition as described above in an amount that is sufficient to inhibit the growth of organisms forming body odor at the site of the application, in particular of *Corynebacterium xerosis,*
The cosmetically and/or pharmaceutically acceptable excipients or carriers can be blended with the composition of the invention each alone or as a combination of two or more of them and thus a characteristic body care product, preferably a deodorant is prepared.

By way of example, the following excipients or carriers are mentioned:
*Antihydrotics, antiperspirants, antitranspirants:*
The pharmaceutical or cosmetic composition according to the invention may also further comprise at least one further so-called antihydrotics, antiperspirants or *antitranspirants*, such as aluminium compounds, such as aluminium sulfate or aluminium chlorohydrate, zinc salts and citric acid compounds.

### Fragrances:

The pharmaceutical or cosmetic composition according to the invention may also further comprise at least one fragrance and/or at least one coloring agent. Fragrances and/or coloring agents well known to those skilled in the art may be used in effective amounts to impart the desired fragrance and color to the compositions of the invention.

Any suitable perfumes may be used with no particular restriction which may be either synthetic perfumes or natural essential oils. The fragrance, for examples, include hydrocarbons, alcohols, phenols, aldehydes, and/or acetals, ketones and/or ketals, ethers, synthetic musks, acids, lactones, esters, halogen-containing compounds, and natural perfumes.

Specific examples hydrocarbons perfumes such as limonene, pinen, y-terepinen, and caryophyllene; alcohol perfumes such as phenyl ethyl alcohol, terepineol, bacdanol, geraniol, nerol, linarol, and cis-3-hexenol; aldehyde perfumes such as lilial, citral, aldehyde C-8, aldehyde C-9, aldehyde C-II, hexyl cynnamic aldehyde, vanillin, and heliotropin; keton perfumes such as yonon, rosephenone, woody flow, damasnin, isoe super; other perfume such as musks, eugenol and coumarin, in which compounds containing no sulfur or nitrogen atom are especially preferable; essential oils such as lemon oil, orange oil, and peppermint oil; and essences such as apple essence and strawberry essence. In is to be understood that, in case a fragrance is used, one or more fragrances may be used..

### Diluent:

According to one embodiment of the invention, the composition according to the invention comprises at least one diluent, e.g. purified water.

### Emulsifier:

Optionally, the pharmaceutical or cosmetic composition according to the invention comprises one or more emulsifiers. In particular, the emulsifiers (i.e., emulsifying agents) are preferably used in amounts effective to provide uniform blending of ingredients of the composition.

### Preferred emulsifiers include one or more of

(i) anionics such as fatty acid soaps, e.g., potassium stearate, sodium stearate, ammonium stearate, and triethanolamine stearate; polyol fatty acid monoesters containing fatty acid soaps, e.g., glycerol monostearate containing either potassium or sodium salt; sulfuric esters (sodium salts), e.g., sodium lauryl 5 sulfate, and sodium cetyl sulfate; and polyol fatty acid monoesters containing sulfuric esters, e.g., glyceryl monostearate containing sodium lauryl surfate;
(ii) cationics chloride such as N(stearoyl colamino formylmethyl) pyridium; N-soya-N-ethyl morpholinium ethosulfate; alkyl dimethyl benzyl ammonium chloride; diisobutylphenoxytheoxyethyl dimethyl benzyl ammonium chloride; and cetyl pyridium chloride; and
(iii) nonionics such as polyoxyethylene fatty alcohol ethers, e.g., monostearate; polyoxyethylene lauryl alcohol; polyoxypropylene fatty alcohol ethers, e.g., propoxylated oleyl alcohol; polyoxyethylene fatty acid esters, e.g., polyoxyethylene stearate; polyoxyethylene sorbitan fatty acid esters, e.g., polyoxyethylene sorbitan monostearate; sorbitan fatty acid esters, e.g., sorbitan; polyoxyethylene glycol fatty acid esters, e.g., polyoxyethylene glycol monostearate (such as Tagat S2); and polyol fatty acid esters, e.g., glyceryl monostearate and propylene glycol monostearate; and ethoxylated lanolin derivatives, e.g., ethoxylated lanolins, ethoxylated lanolin alcohols and ethoxylated cholesterol. The selection of emulsifiers is exemplarly described in Schrader, Grundlagen und Rezepturen der Kosmetika, Hüthig Buch Verlag, Heidelberg, 2^{nd} edition, 1989, 3^{rd} part.

### Film Former:

Optionally the pharmaceutical or cosmetic composition according to the invention may comprise a film former. In general, film formers which are used in accord with the invention preferably keep the composition smooth and even. Such formers include, but are not limited to, one or more of the following: acrylamide/sodium acrylate copolymer; ammonium acrylates copolymer; Balsam Peru; cellulose gum; ethylene/maleic anhydride copolymer; hydroxyethylcellulose; hydroxypropylcellulose; polyacrylamide; polyethylene; polyvinyl alcohol; pvm/MA copolymer (polyvinyl methylether/maleic anhydride); PVP (polyvinylpyrrolidone); maleic anhydride copolymer such as PA-18 available from Gulf Science and Technology; PVP/hexadecene copolymer such as Ganex V-216 available from GAF Corporation; and acryliclacrylate copolymer.

### Waxes:

Optionally the pharmaceutical or cosmetic composition of the present invention comprises one or more waxes. Preferred waxes include one or more of the following: animal waxes, such as beeswax, and preferably hexadecanoic acid ester of tricontanol contained therein, spermaceti, or wool wax (lanolin); plant waxes, such as carnauba or candelilla; mineral waxes, such as montan wax or ozokerite; and petroleum waxes, such as paraffin wax and microcrystalline wax (a high molecular weight petroleum wax). Alternatively or in addition to these, one or more synthetic waxes may be used in the composition, wherein said one or more synthetic waxes preferably include polyethylene, polyoxyethylene, and hydrocarbon waxes derived from carbon monoxide and hydrogen, and combinations of two or more thereof.

### Antioxidant

The pharmaceutical or cosmetic composition comprises an antioxidant, e.g. butylhydroxyanisol.

### Further antibacterial agents

Optionally the pharmaceutical or cosmetic composition comprises further antibacetrail agents, such as benzoic acid, sodium benzoate, isopropyl p-hydroxybenzoate, isobutyl p-hydroxybenzoate, ethyl p-hydroxybenzoate, methyl p-hydroxybenzoate, butyl p-hydroxybeazoate, propyl p-hydroxybenzoate, sodium sulfite, sodium hyposulfite, potassium pyrosulfite, sorbic acid, potassium sorbate, sodium dehydroacetate, thujaplicin, udo extract, storax extract, wild tansy extract, milt protein extract, and zymolytic Yokuinin extract.

According to a preferred embodiment of the invention, the pharmaceutical or cosmetic composition, preferably the deodorant composition, comprises at least one compound selected from the group consisting of glyceryl monostearate, cetyl alcohol, PEG and water.

### Surfactants:

According to a further preferred embodiment, the pharmaceutical or cosmetic composition according to the invention comprises at least one surfactant such as anionic, cationic, non-ionic, amphoteric surfactants and mixtures thereof. Suitable surfactants for use in cosmetical and pharmaceutical compositions are well known in the art. Surfactants are amphiphilic substances which are able to dissolve organic, non-polar substances in water. The hydrophilic components of a surfactant molecule are mostly polar functional groups, while the hydrophobic portions are generally non-polar hydrocarbon residues. Surfactants are generally classified according to the type and charge of the hydrophilic molecule portion. A distinction can be made between four groups, which have already been mentioned above, that is anionic surfactants, cationic surfactants, amphoteric surfactants and non-ionic surfactants. Anionic surfactants usually contain carboxylate, sulfate or sulfonate groups as functional groups. Cationic surfactants are almost exclusively characterized by the presence of a quaternary ammonium group. In aqueous solution they form positively charged organic ions in an acidic or neutral medium. Amphoteric surfactants contain both anionic and cationic groups and accordingly behave in aqueous solution like anionic or cationic surfactants, depending on the pH value. Typical examples for non-ionic surfactants are poly ether chains.

For example, the composition may comprise a surfactant such steareth-2 or steareth-20.

### Dental Care:

According to a further preferred embodiment of the invention the cosmetic and/or pharmaceutical composition is used for dental care or in a dental care product.

Preferably the present invention also relates to a pharmaceutical or cosmetic composition, as described above, comprising one or more cosmetically and/or pharmaceutically acceptable carriers and/or excipients for use in treating or preventing dental caries. Further, a method of treating dental caries using a pharmaceutical or cosmetic composition, as described above, comprising one or more cosmetically and/or pharmaceutically acceptable carriers and/or excipients is described.

Examples of suitable oral care products in which the cosmetic and/or pharmaceutical composition can be used include, but are not limited to dentifrices (e.g., toothpaste, toothpaste gels, toothpowders, denture cleaning agents and compounds, mouthwashes and mouth rinses, toothpicks, dental floss, chewing gums, pastilles, lozenges, dissolvable tablets, chewable tablets, etc.). Such products are e.g. described in US 2008/0274063.

Thus, the present invention also relates a dentrifice, in particular, a toothpaste, tooth gel, mouthwash or mouthrinse, comprising a pharmaceutical or cosmetic composition, as described above, preferably consisting of a pharmaceutical or cosmetic composition, as described above..

According to one preferred embodiment, the present invention thus relates to a toothpaste or toothgel consisting of a pharmaceutical or cosmetic composition as described above, said toothpaste preferably comprises are least one excipient selected from the group consisting of binders, thickeners, flavoring agents, sweetening agents, antioxidants, fluorides, preservatives, pH adjusting agents, desensitizing agents, stabilizing agents and gelling agents.

### Binder:

According to a preferred embodiment, the pharmaceutical or cosmetic composition is provided as a toothpaste or toothgel, and comprises at least one binder. Examples for suitable binders include, but are not limited to, carrageenan and natural gums such as gum karaya, xanthan gum, gum Arabic and gum tragacant.

Suitable thickeners are e.g. magnesium aluminium silicate or silica.

### Sweetener and flavoring agents

The pharmaceutical or cosmetic composition may further comprises at least one at least one sweetener and/or flavoring agent. Examples of suitable sweeteners and flavoring agents, are, e.g. agents comprising oils or extracts derived from plants and fruits such as citrus oils, fruit essences, mint, peppermint oil, spearmint oil, clove oil, oil of wintergreen, anise, sassafras, sage, eucalyptus, marjoram, cinnamon, lemon, orange, banana, cherry, fennel, apple; pineapple, grape, strawberry and blueberry. Sweeteners include, but are not limited to, xylitol, glycerol, sorbitol, maltitol, erythritol, sucrose, lactose, dextrose, maltose, dextrin, fructose, galactose and the like.

### Diluent

The pharmaceutical or cosmetic composition may further comprises at least a diluent, preferably a water-glycerin solution and may additionally comprise one or more of flavoring agent, humectant, sweetener, emulsifier (e.g., poloxamer) and coloring agents. Regarding suitable examples for sweetener, flavoring agent and emulsifier, reference is made to the examples present above.

### Humectant

Suitable humectant compounds include propylene glycol, glycerol, sorbitol, mannitol, corn syrup, 3-cyclodextrin, amylodextrin, and the like.

In the following, by way of example, the following especially preferred embodiments of the invention are mentioned:

### Thickening agents

The pharmaceutical or cosmetic composition according to the invention may also further comprise at least one suitable thickener. Suitable thickeners are the swelling agents customarily used for gel formation in galenic pharmacy.

Examples of suitable thickeners include natural organic thickeners, such as agar-agar, gelatin, gum arabic, a pectin, and the like, modified organic natural compounds, such as carboxymethylcellulose or cellulose ethers, or fully synthetic organic thickeners, such as poly arylic compounds, vinyl polymers, or poly ethers.

In some embodiments, the excipient can increase the smoothness or other properties of the composition. Such additives include, but are not limited to glycerin, propylene glycol, butylene glycol, esters, diacyl glycerol esters, and starch.

Furthermore, the thickeners may be selected from algin; carbomers cellulose gum; cetearyl alcohol, cocamide DEA, dextrin; gelatin; hydroxyethylcellulose; hydroxypropylcellulose; hydroxypropyl methylcellulose; magnesium aluminum silicate; myristyl alcohol; oat flour; oleamide DEA; oleyl alcohol; PEG-7M; PEG-14M; PEG-9OM; stearamide DEA; stearamide MEA; stearyl alcohol; tragacanth gum; wheat starch; xanthan gum; wherein DEA is diethanolamine, and MEA is monoethanolamine. Alternatively or in addition thereto, thickeners used the composition of the present invention may comprise one or more of aluminum stearates; beeswax; candelilla wax; carnauba; ceresin; cetearyl alcohol; cetyl alcohol; cholesterol; hydrated silica; hydrogenated castor oil; hydrogenated cottonseed oil; hydrogenated soybean oil; hydrogenated tallow glyceride; hydrogenated vegetable oil; hydroxypropyl cellulose; lanolin alcohol; myristyl alcohol; octytdodecyl stearoyl sulfate; oleyl alcohol; ozokerite; microcystalline wax; paraffin, pentaerythrityl tetraoctanoate; polyacrylamide; polybutene; polyethylene; propylene glycol dicaprylate; propylene glycol dipelargonate; stearalkonium hectorite; stearyl alcohol; stearyl stearate; synthetic beeswax; trihydroxystearin; trilinolein; tristearin; zinc stearate; and the like.

According to a preferred embodiment of the invention, the composition according to the invention comprises at least one thickener, such as glycerol or xanthane gum.

### Chelating agents

The pharmaceutical or cosmetic composition according to the invention may also further comprise at least one chelating agents, i.e. an agent that may bind metal ions or metallic compounds. Preferred chelating agents include tetrasodium- and trisodium-ethylenediaminetetraacetate, sodium phytate and the like.

### Figures:

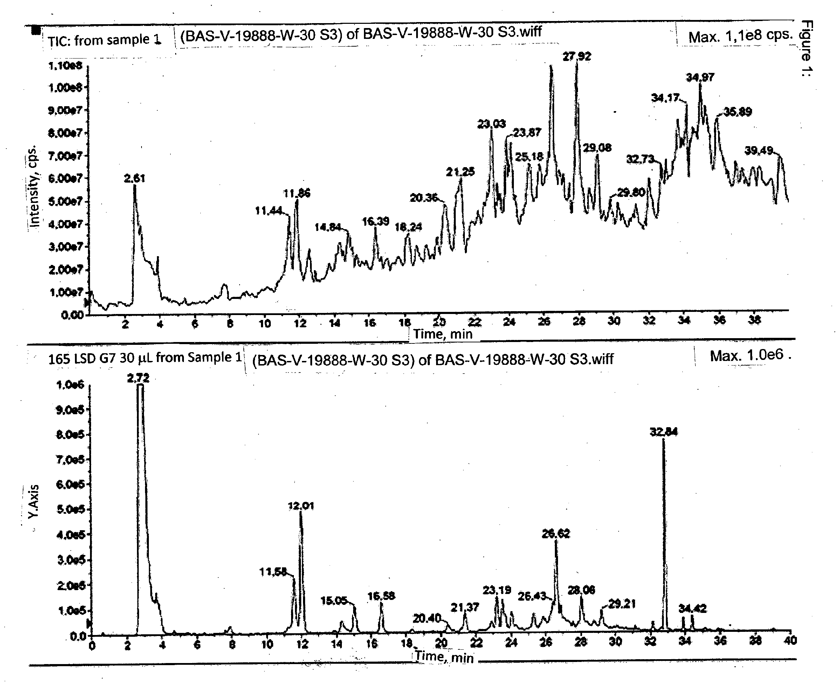

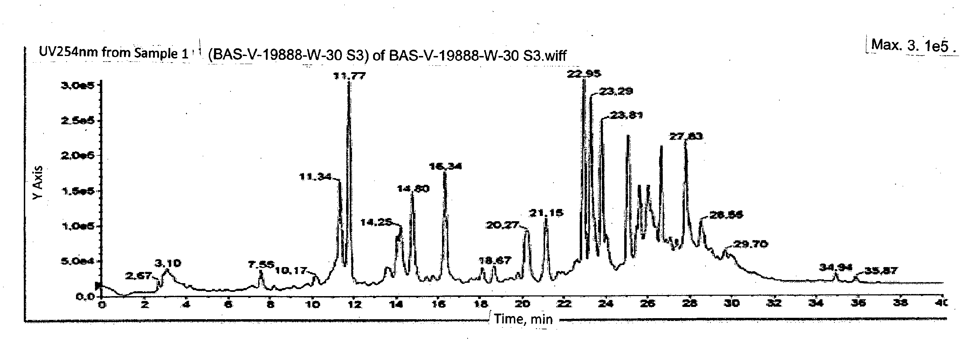

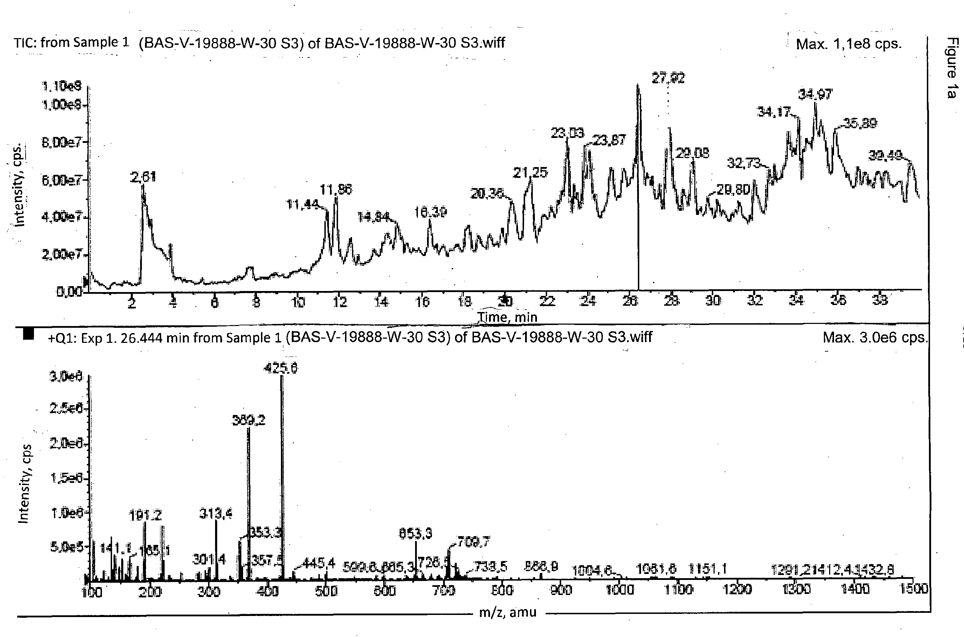

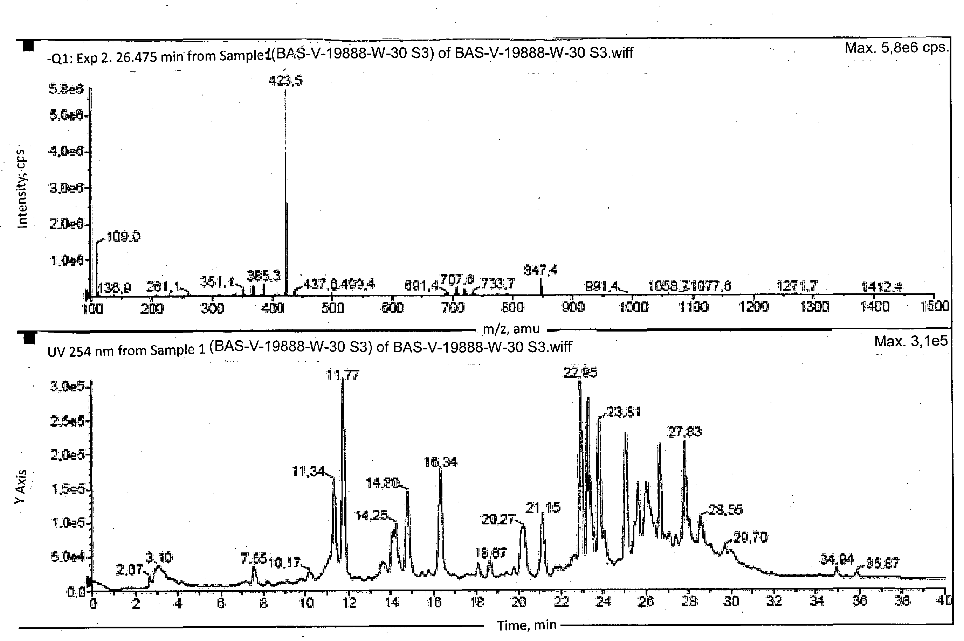

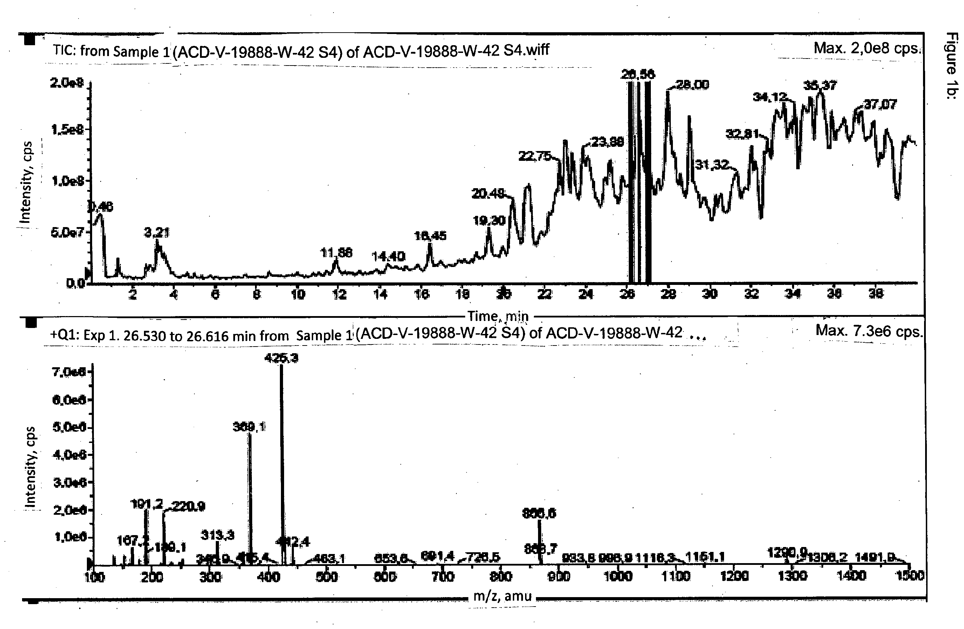

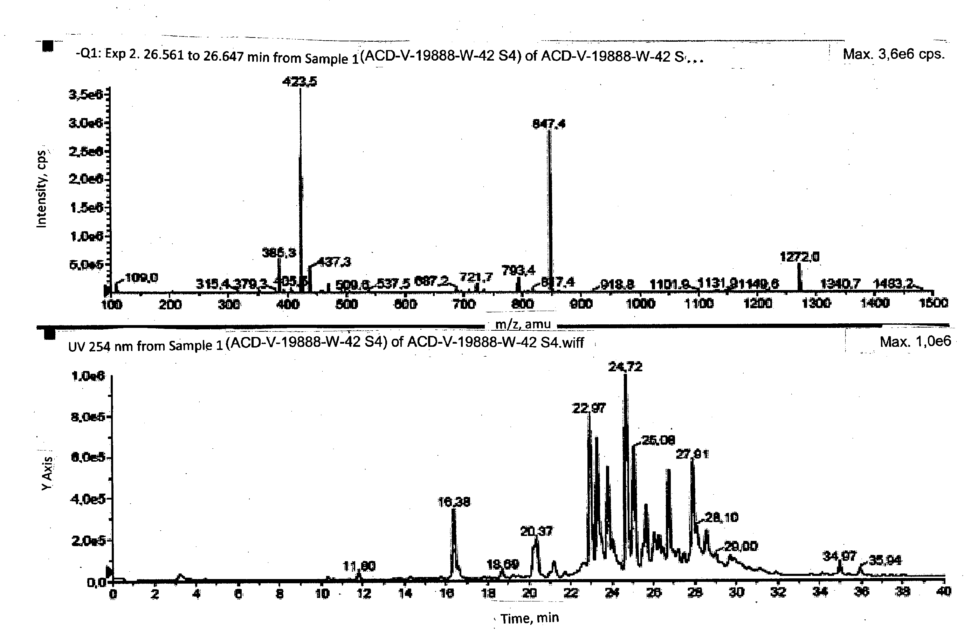

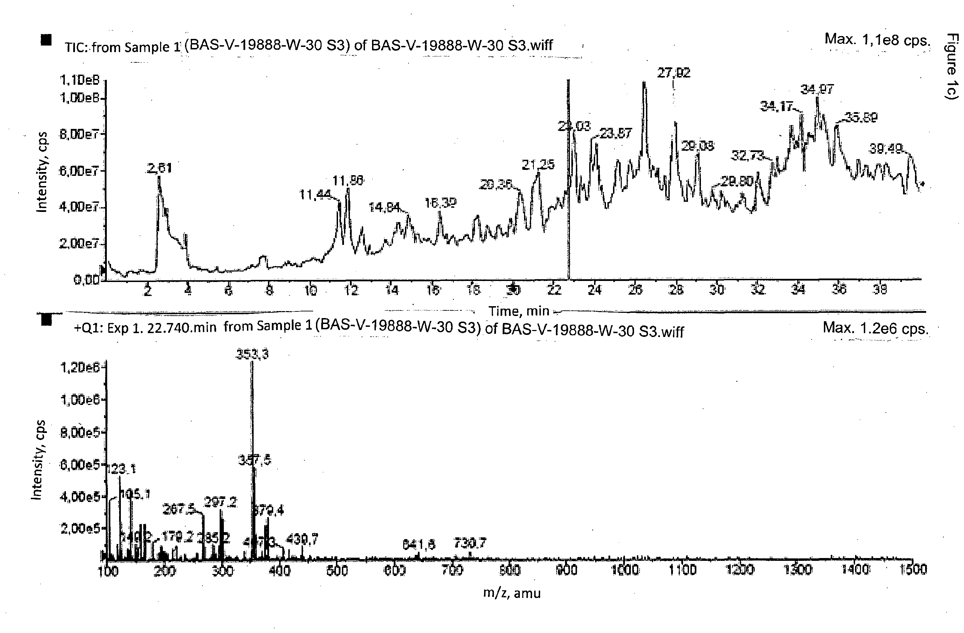

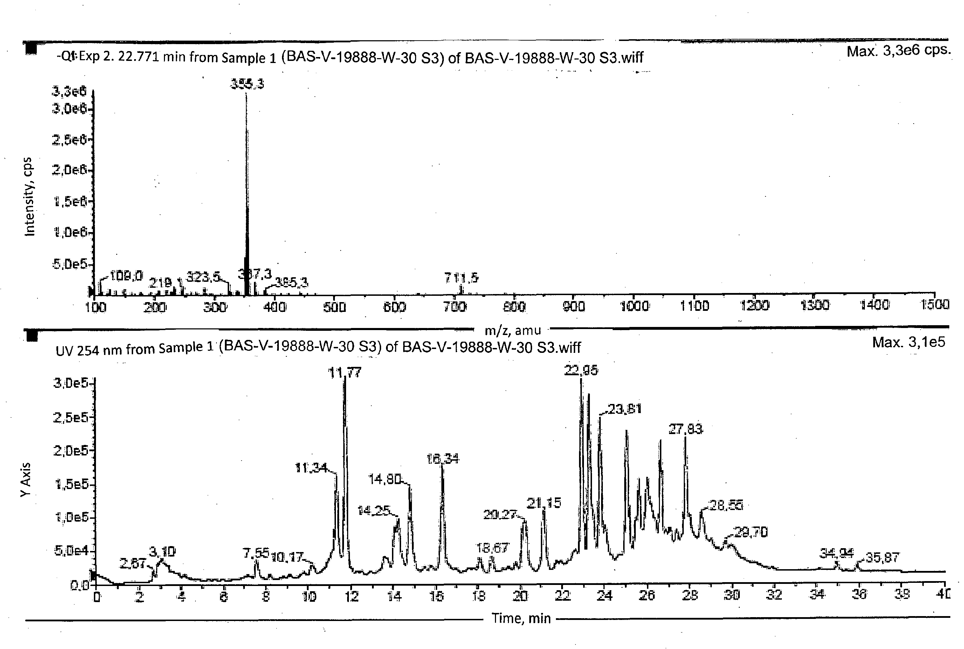

**Figure 1****:** HPLC chromatograms of the extract obtained according to example 1, measured according to the general procedure I.
**Figure 1a****:** HPLC chromatograms of the extract obtained according to example 1 and corresponding ESI spectra of the peak detected at 26.4 min (Detection:TIC, (+)-ESI, (-)-ESI, UV 254 nm): licoricidine (molecular weight 424 g/mol)

| ionisation | m/z | interpretation |
|---|---|---|
| (+)-ESI | 425 | [M+H]⁺ |
| (-)-ESI | 423 | [M-H]⁻ |

**Figure 1b**: HPLC chromatograms of the extract obtained according to example 1 and corresponding ESI spectra of one of the peaks at retention time between 25.4 and 26.4min (Detection:TIC, (+)-ESI, (-)-ESI, UV 254 nm): glyasperin D (molecular weight 370 g/mol),
**Figure 1c**: HPLC chromatograms of the extract obtained according to example 1 and corresponding ESI spectra of the peak with a retention time of 22.7 min (Detection:TIC, (+)-ESI, (-)-ESI, UV 254 nm): glyasperine C (molecular weight 356 g/mol)

| ionisation | m/z | interpretation |
|---|---|---|
| (+)-ESI | 357 | [M+H]⁺ |
| (-)-ESI | 355 | [M-H]⁻ |

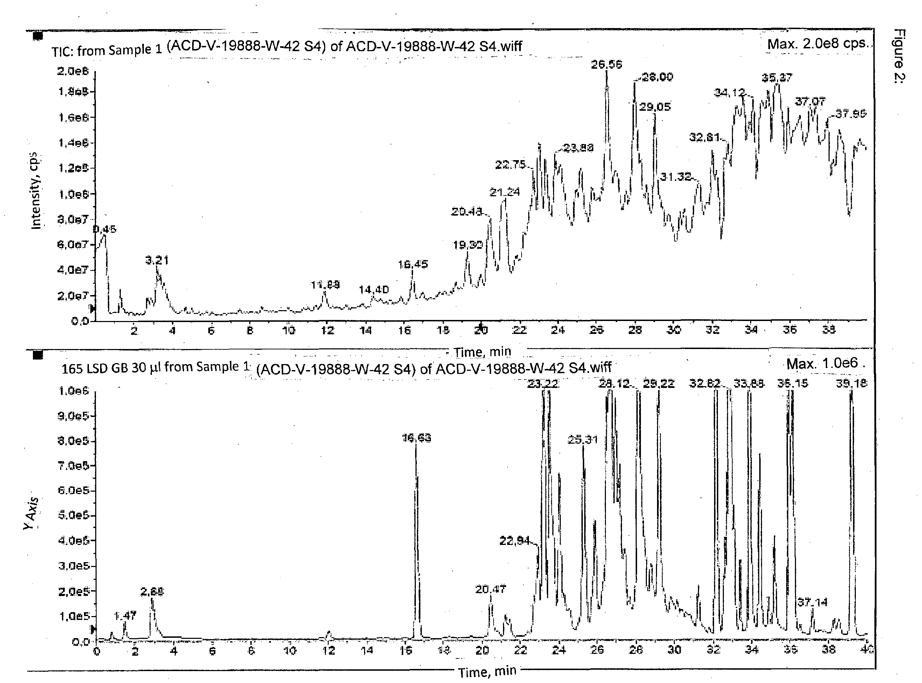

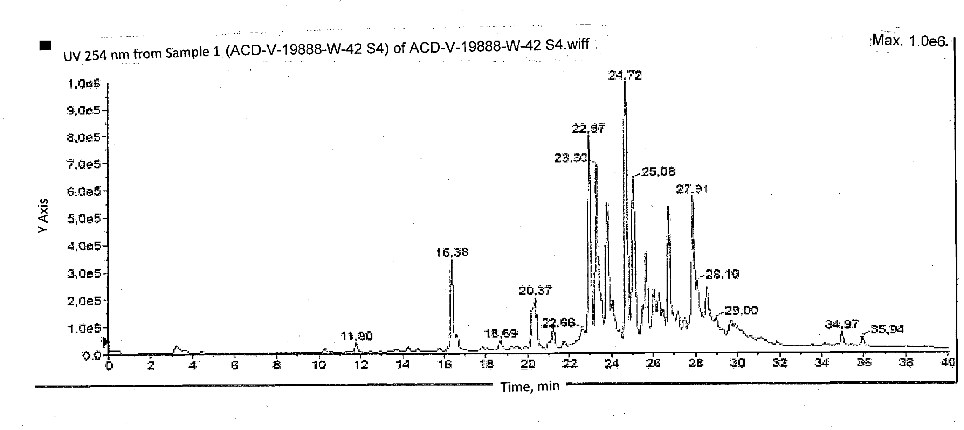

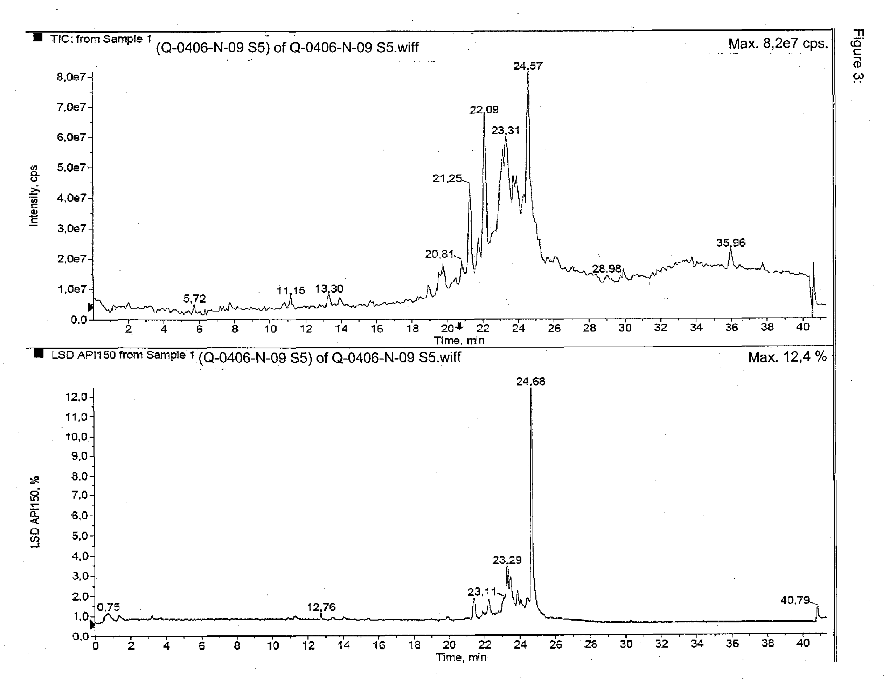

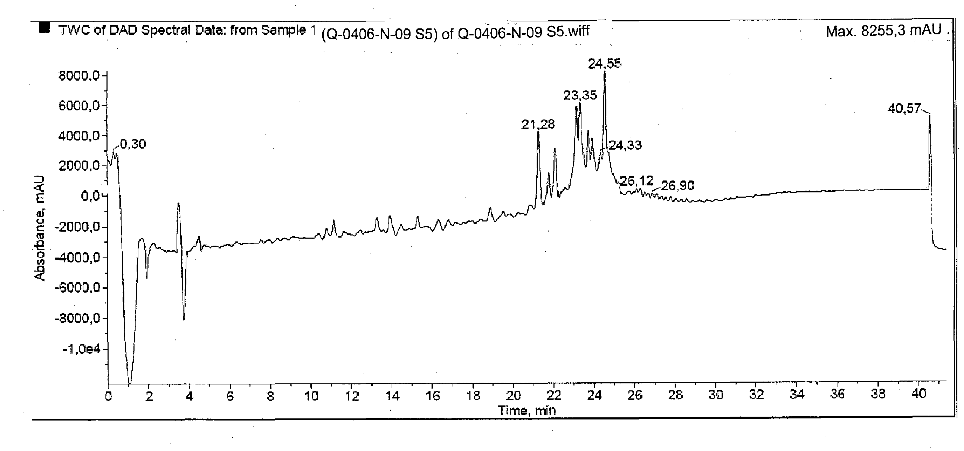

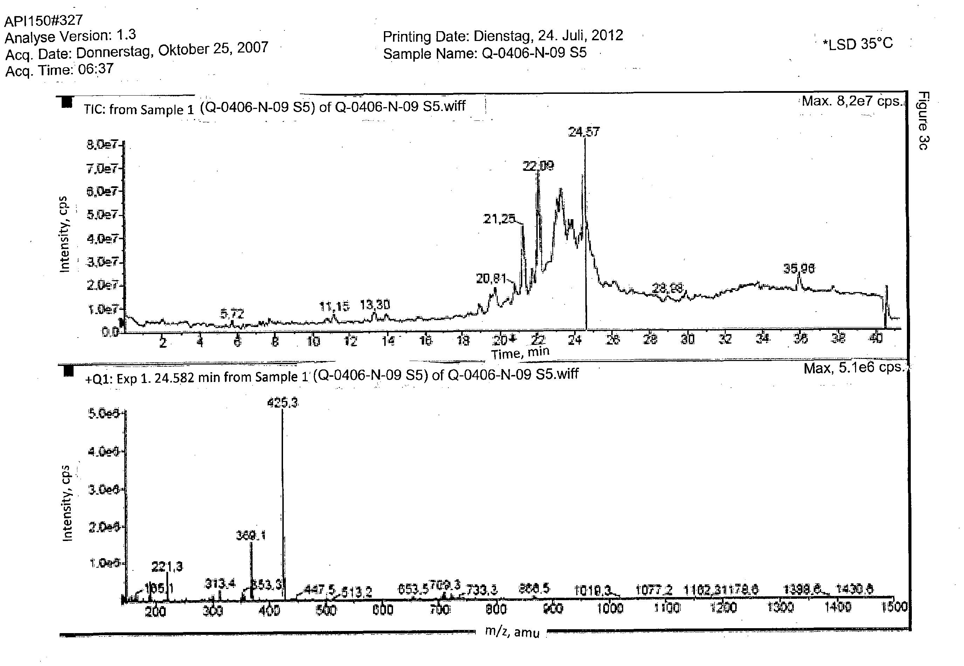

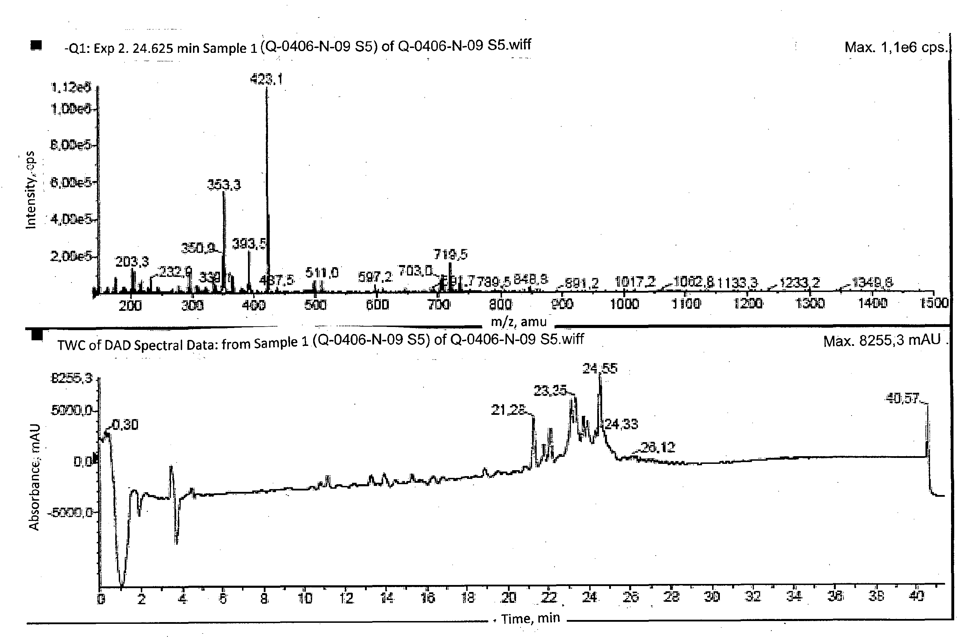

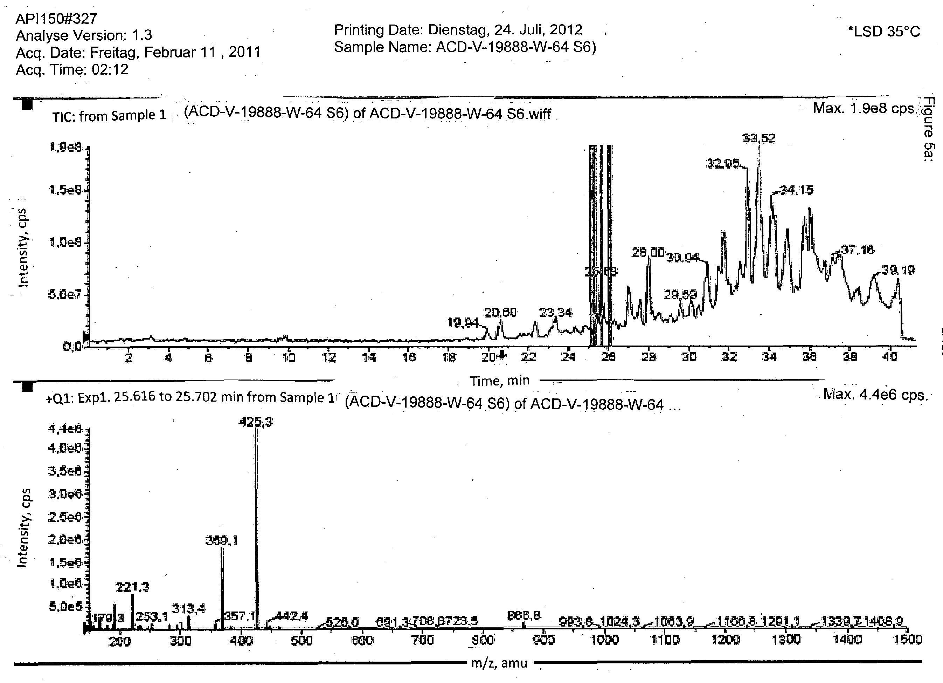

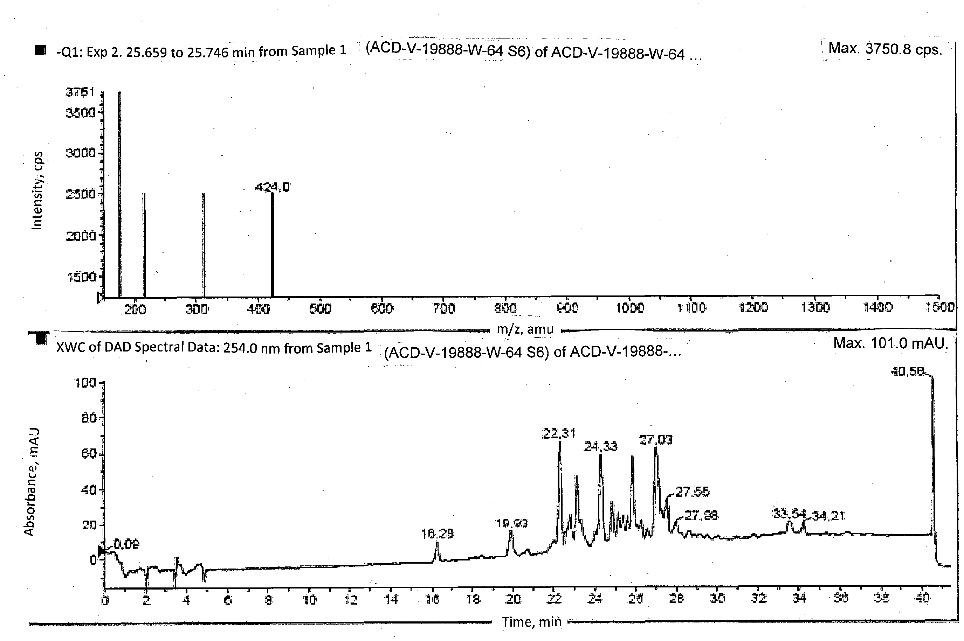

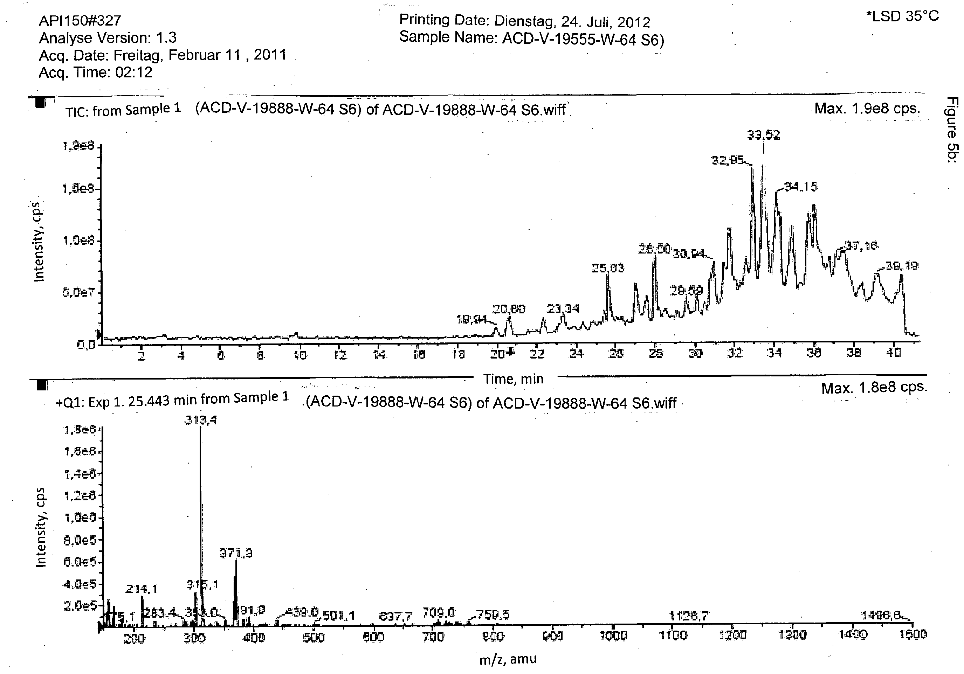

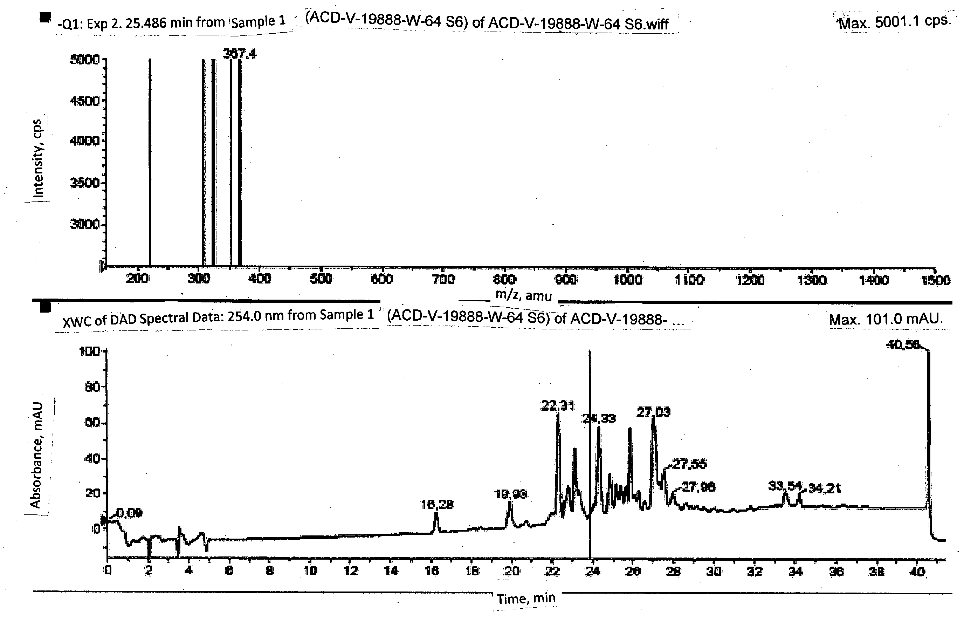

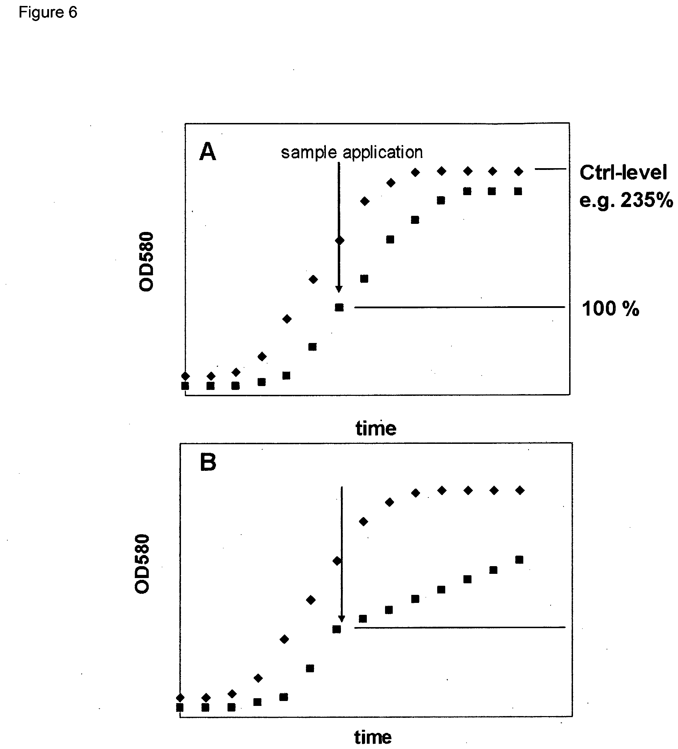

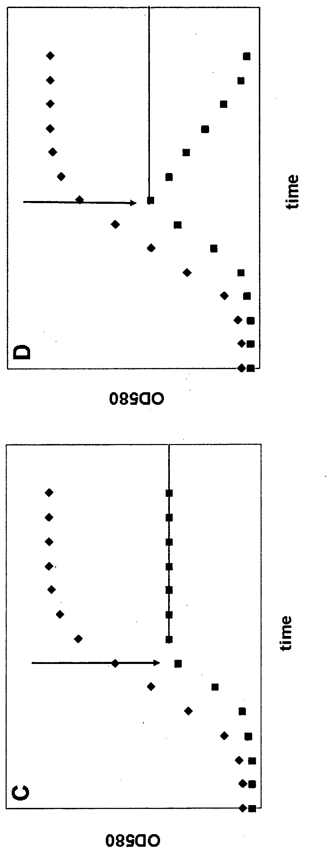

gancaonin I (molecular weight 354 g/mol), one of the peaks at retention time between 26.4 and 27.2min
glycyrrhisoflavone (molecular weight 354 g/mol), one of the peaks at retention time between 22.5and 23.5min
**Figure 2****:** HPLC chromatograms of the extract obtained according to example 2, measured according to the general procedure I.
The respective peak at retention time measured for the active components were as follows
licoricidine (molecular weight 424 g/mol) 26.6 min
glyasperin D (molecular weight 370 g/mol), one of the peaks at retention time between 25.4 and 26.6min
glyasperine C (molecular weight 356 g/mol), one of the peaks at retention time between 22 and 23min
gancaonin I (molecular weight 354 g/mol),, one of the peaks at retention time between 26.6 and 27.5min
glycyrrhisoflavone (molecular weight 354 g/mol), one of the peaks at retention time between 22.5and 23.5min
**Figure 3****:** HPLC chromatograms of the extract obtained according to example 5, measured according to the general procedure I.
The respective peak at retention time measured for the active components were as follows
licoricidine (molecular weight 424 g/mol) 24.6 min (see figure 3c)
glyasperin D (molecular weight 370 g/mol), one of the peaks at retention time between 24.2 and 25.2min
glyasperine C (molecular weight 356 g/mol), one of the peaks at retention time between 21.2 and 22.2min
gancaonin I (molecular weight 354 g/mol),, one of the peaks at retention time between 24.6 and 25.5min
glycyrrhisoflavone (molecular weight 354 g/mol), one of the peaks at retention time between 21.5 and 22.5min
**Figure 3c****:** HPLC chromatograms of the extract obtained according to example 5, and corresponding ESI spectra of the peak with a retention time of 24.6 min.
**Figure 4a** **and** **4b****:** HPLC chromatograms of the extract obtained according to example 3 (maceration)
The respective peak at retention time measured for the active components were as follows
licoricidine (molecular weight 424 g/mol) 25.6 min
glyasperin D (molecular weight 370 g/mol), one of the peaks at retention time between 25 and 25.7min
glyasperine C (molecular weight 356 g/mol) 22.2 min
gancaonin I (molecular weight 354 g/mol), one of the peaks at retention time between 25.5 and 26.5min
glycyrrhisoflavone (molecular weight 354 g/mol), one of the peaks at retention time between 22 and 23min
**Figure 5a** **and** **5b****:** HPLC chromatograms of the extract obtained according to example 4 (co-pressing) and corresponding ESI spectra of the peak with a retention time of 25.6 min (5a)and 25.5 min (5b) peak at retention time
licoricidine (molecular weight 424 g/mol) 25,6 min
glyasperin D (molecular weight 370 g/mol) 25,4 min
glyasperine C (molecular weight 356 g/mol), one of the peaks at retention time between 22 and 23min
gancaonin I (molecular weight 354 g/mol), one of the peaks at retention time between 25.5 and 26.5min
glycyrrhisoflavone (molecular weight 354 g/mol), one of the peaks at retention time between 22 and 23min
**Figure. 6****: Schematic drawing of the mid-log assay and corresponding results.** Cells are cultivated to half-maximal CV580 (biofilm formation) before test compounds are added (arrow). Value of CV580 and OD580 at time of sample application is set 100%, respectively. **A)** ineffective test compound show no inhibition of growth. / biofilm. Level-% is identical to control-level-%; **B)** test compound inhibits cell growth / biofilm formation, level-% < Ctrl-level, > 100 %; **C)** stagnation; no further growth / biofilm formation upon addition of test compound, level-% = 100%; **D)** cell-lysis or biofilm detachment; CV580 or OD580 diminishes upon addition of test compound; level-% < 100%. Diamonds, untreated control cells; rectangles, cells treated with test compounds.

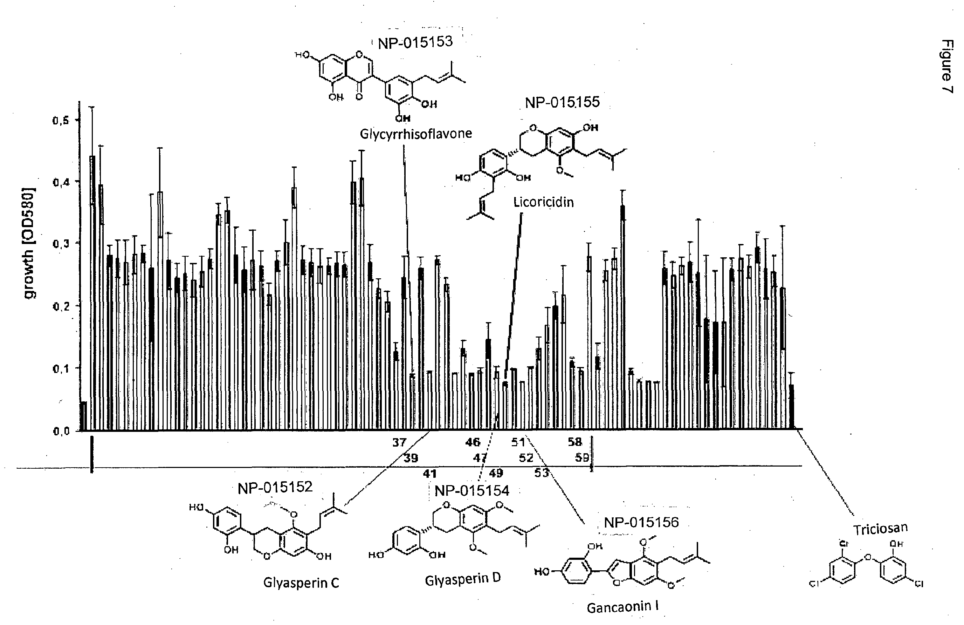

**Figure. 7****:** Identification of the active components, shown is the growth inhibition determined as OD 580 for selected compounds optical density at 580nm

The following examples are intended to illustrate the present invention without limiting it. Unless indicated otherwise, all amounts, parts and percentages are based on the weight and the total amount, or on the total weight and the total amount, or on the total weight of the preparations.

### Examples

Glyhzyrrhiza pallidiflora was obtained from Hyphagenesis, Japan, # HYL-Nr.34

### Example 1: Preparation of a Glycyrrhiza pallidiflora extract by extraction with ethanol:

The dried and finely grounded plant material (*Glycyrrhiza pallidiflora*) were extracted at room temperature twice with fivefold ethanol in total (sonicated for about 15 minutes, shaken for about 30 minutes and afterwards centrifugated, yielding approx. 6% extract regarding the quantity of plant material.

### Example 2 Preparation of a Glycyrrhiza pallidiflora extract by extraction with isopropane alcohol/n-heptane

The dried and finely grounded plant material *(Glycyrrhiza pallidiflora)* were extracted at room temperature twice with fivefold solvent (isopropane alcohol/n-heptane, 10:90) in total (sonicated for about 15 minutes, shaken for about 30 minutes and afterwards centrifugated, yielding approx. 1,5% extract regarding the quantity of plant material.

### Example 3 Preparation of a Glycyrrhiza pallidiflora extract by maceration using safflower oil

The dried and finely grounded plant material *(Glycyrrhiza pallidiflora)* were extracted with threefold safflower oil for three days at 50°C with stirring occasionally and afterwards centrifugated, yielding approx. 50% maceration oil regarding the added quantity of safflower oil.

### Example 4 Preparation of a Glycyrrhiza pallidiflora extract co-pressing procedure

The dried and coarsely grounded plant material (*Glycyrrhiza pallidiflora*, 6 to 8 mm particle size) in addition of threefold quantity of peeled sunflower seed were processed by the SPE (Short-Press-Extraction)-technology (see US 2002/0028272), yielding approx. 50 to 60 % received essential oil regarding the added quantity of peeled sunflower seed.

### Example 4A: Preparation of a Glycyrrhiza pallidiflora extract by extraction with methyl-tert-buthylether)/methanol

The dried and finely grounded plant material *(Glycyrrhiza pallidiflora)* were extracted at room temperature twice with fivefold amount of solvents MTB (methyl-tert-buthylether)/methanol (1:1), 2x methanol). The mixture was sonicated for about 15 minutes, shaken for about 30 minutes and afterwards centrifugated, room temperature)

### 1.) Fractionation of the extracts

The extracts obtained were fractionated by RP (reversed-phase) HPLC yielding twelve (12) fractions.

Sample preparation: 1g extract were dissolved in 3ml DMSO, 1ml water were added and filtrated, liquid injection of the filtrate.

### Method description:

Column: SelectB 12µm 50x25mm with pre-column
Detection: HPLC-ELSD-UV (254nm)
Mobile Phase: A: H2O; B: ACN:MeOH (1:1)
Gradient: 10% B 0.5 min; from 10% B to 100% B in 0.8 min, 6.3 min 100%B
Flow: 28 ml/min

### 2.) MS-analysis of the extracts

**LC/MS-Method**

| | |
|---|---|
| Column: | LiChrospher 60 RP select B 5µm, 250x4 mm |
| | and pre-column 4x4mm |
| Mobile Phase: | A: 5mM ammonium formiate and 0.1% formic acid |
| | B: methanol:acetonitrile (1:1) with 5mM ammonium formiate and 0.1% formic acid |
| Detection: | ELSD (Sedex75, pressure 3.5bar, 35°C), DAD 210-400 nm |
| Gradient: | 15% B to 100%B in 30min, 10min 100%B |
| Flow Rate: | 0.9 ml/ min |
| Scan Range: | 150-1500 amu, pos/neg switch |

### Example 5 : Stability assessment of licoricidine using different stabilizers

### Example 5.1 Stability assessment of licoricidine in aqueous solution with 0.1% ethanolic extract

Five times 5 mg ethanolic extract (0.1%) were dissolved in 1.5 ml ethanol and 3.5 ml buffer pH 7.
- To the first batch 20mg citric acid (0.4%) were added as a stabilizer and the resulting batch was stored at 40°C.
- To the second batch 50mg citric acid (1.0%) were added as a stabilizer and the resulting batch was stored at 40°C.
- To the third batch 20mg ascorbic acid (0.4%) were added as a stabilizer and the resulting batch was stored at 40°C.
- To the fourth batch 50mg ascorbic acid (1.0%) were added as a stabilizer and the resulting batch was stored at 40°C.
- The fifth batch was stored as a reference at -18°C.

### Example 5.2 Stability assessment of licoricidine in safflower oil with 0.1% lipophilic extract

30 mg lipophilic extract were dissolved in 30 ml safflower oil at 80°C.

The obtained solution was split into three batches:
- To the first batch 10 mg/ml ascorbic acid palmitate were added as a stabilizer and the resulting batch was stored at 40°C.
- To the second batch 10 mg/ml BHA (Butylhydroxyanisol) were added as a stabilizer and the resulting batch was stored at 40°C.
- The third batch was stored as a reference at -18°C.

### Example 5.3 Stability assessment of licoricidine in safflower oil with 0.4% lipophilic extract

Dissolve 120 mg lipophilic extract in 30 ml safflower oil at 80°C.

Split in tree (3) batches:
- Add to the first batch 10 mg/ml ascorbic acid palmitate as a stabilizer and store at 40°C.
- Add to the second batch 10 mg/ml BHA (Butylhydroxyanisol) as a stabilizer and store at 40°C.
- Freeze the third batch as a reference at -18°C.

### Example 5.4 Stability assessment of licoricidine in the maceration oil

- Add to 10 ml maceration oil 100 mg ascorbic acid palmitate as a stabilizer and store at 40°C.
- Add to 10 ml maceration oil 100 mg BHA (Butylhydroxyanisol) as a stabilizer and store at 40°C.
- Freeze 10 ml maceration oil as a reference at -18°C.

### Example 5.5 Analysis of the samples prepared according to examples 5.1 to 5.4

0.5 ml of the respective oil prepared according to any one of examples 5.1 to 5.4 was extracted with 3 ml methanole/water (9:1), centrifugated and the supernatant was used for the analysis.

### (a) The analysis was carried out using the following HPLC method:

Column: Kromasil C18, 125x4mm with pre-column
Detektion: UV (225nm)
Mobile Phase: A: water with 5 mM ammoniumformiate and 0.1 % formic acid;
B: acetonitrile/methanol = 1:1, 5 mM ammoniumformiate and 0.1 % formic acid
Gradient: from 60% B to 80% B in 30min
Flow: 0.8 ml/min
The retention time of the reference peak (licoricidine) is 16.9 (± 0.2) min.

### (b) Results:

The decrease of the peak area of licoricidine (HPLC UV 225nm, retention time approx. 17 min) in an aqueous solution (buffer pH 7 in addition of 30% ethanol) with 0.1% ethanolic extract and 1% citric acid was lower than 20% over eight (8) weeks under storage at 40°C.

The decrease of the peak area of licoricidine (HPLC UV 225nm, retention time 17.0 min) in safflower oil with 0.1% or 0.4% lipophilic extract as well as in maceration oil with addition of 1% BHA (Butylhydroxyanisol) was lower than 20% over twelve (12) weeks under storage at 40°C.

### Example 5A: peroxide content that arise during storage

The obtained compositions were tested for peroxide content and volatile compounds that arise during storage (commonly measured by the rancimat method). The peroxide number is the amount of peroxide in milli-equivalents of active oxygen contained in oil.

POZ = a peroxide rating, value, factor or the numbers for peroxides
POZ (Co-Pressing) 1,98 mmol O2/kg

### Example 6: Studies on the microbial action of a lipophilic extract of Glycyrrhiza pallidiflora (licoricidine = 10 %) against Corynebacterium xerosis

### General test conditions (MIC Value Measurement)

### Assay Principle

*Corynebaterium xerosis* is usually characterized as a rod-like gram positive bacterium. Under laboratory conditions (96 well plates) it takes ∼24 hours until *Corynebaterium xerosis* reaches stationary growth phase. Within this period planktonic growth can be quantified by spectroscopic means and a biofilm can be detected and quantified by standard crystal violet incorporation (c.f. O'Toole & Kolter, 1998, Mol. Microbiol. 28(3), 449-461). Planktonic growth denotes growth of non-adherent cells in the bulk liquid whereas biofilm denotes growth of cells adherent to solid surfaces.

For the screening of potential anti-microbial or anti-biofilm effective compounds a classical assay and the mid-log assay have been used.

### "Classic assay" for susceptibility testing of planktonically growing and biofilm cells

Routine susceptibility testing of *C. xerosis* in high throughput format was done by simultaneous addition of test compounds and inoculum ("classic assay"). Growth of untreated (i.e. no extracts or test compounds added, control) planktonic cells ceased 7 hours after inoculation, and decreased by 13% at 24 hours. Biofilm development reached maximum 10 hours upon inoculation and kept constant for the rest of the test period (30 hours).

Formation of planktonic and biofilm cells was determined 18 - 24 hours after inoculation, thus at a time when some planktonic cells lysed. Data for planktonic proliferation and biofilm formation was collected from identical wells of a microtiterplate. Assays were done as quadruples.

Effects of test compounds were evaluated by comparison of turbidity (optical density, OD580) and crystal violet staining (biofilm; CV580) of treated and untreated samples (control; 100 %).

The classic assay was also the platform to determine dose-response relationships, where serial dilutions of compounds were applied. For description of these relationships, values for the concentration effective in reducing cell proliferation by 50% (IC₅₀) were generated.

### 'Mid-Log assay':

For the so called "mid-log assay", cells are grown to half-maximum biofilm formation before test compounds are added. Due to the focus on biofilm formation, the status / growth phase of planktonically proliferating cells was not taken into account. Relationship between optical density and biofilm development was determined previously, so that the biofilm status of the untreated control could be determined by measuring OD580.

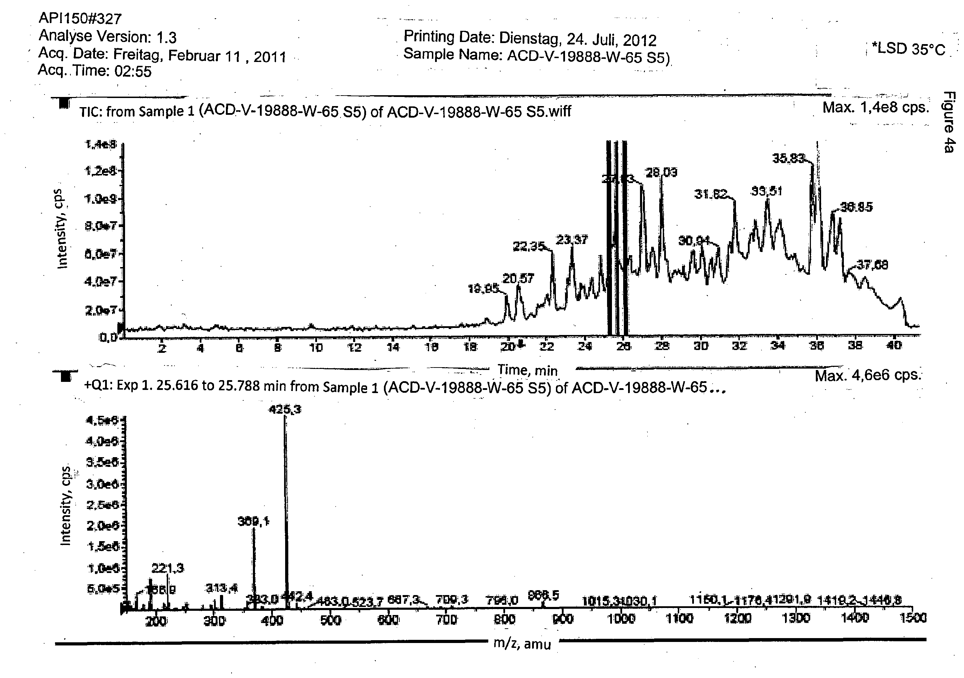

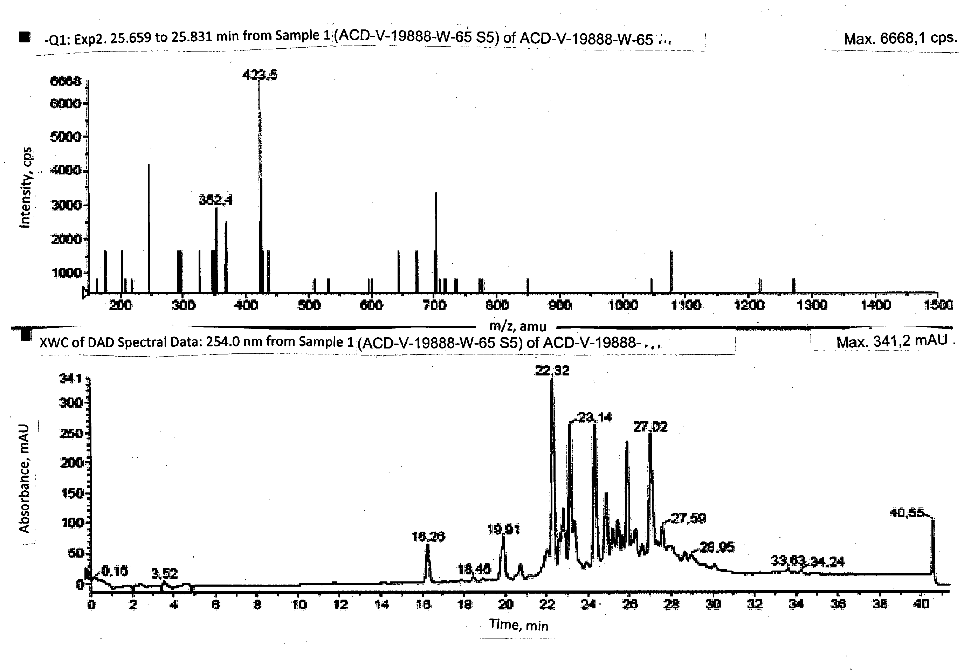

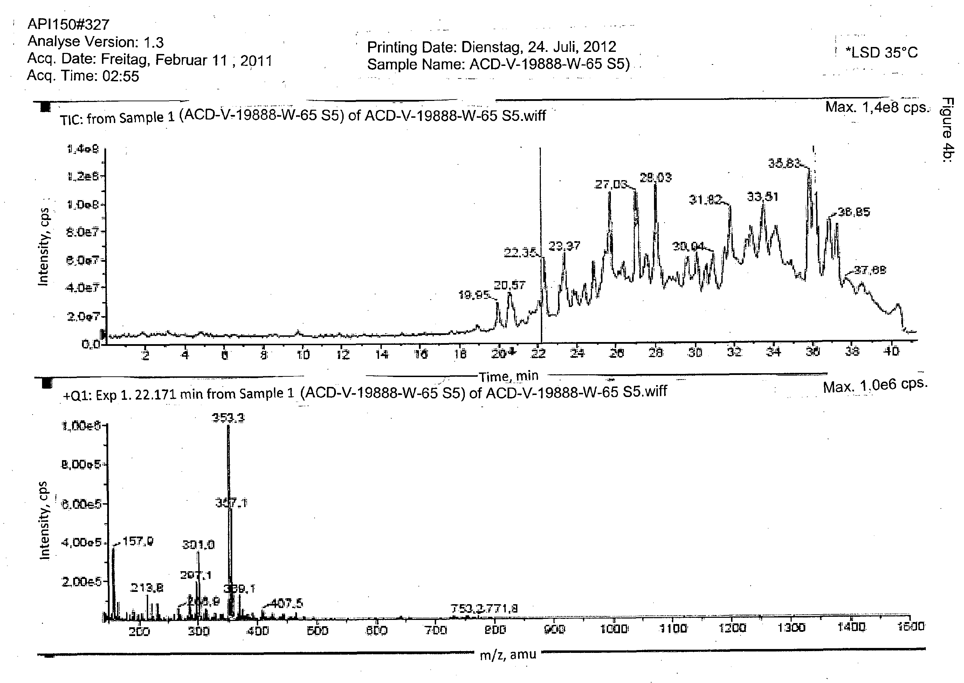

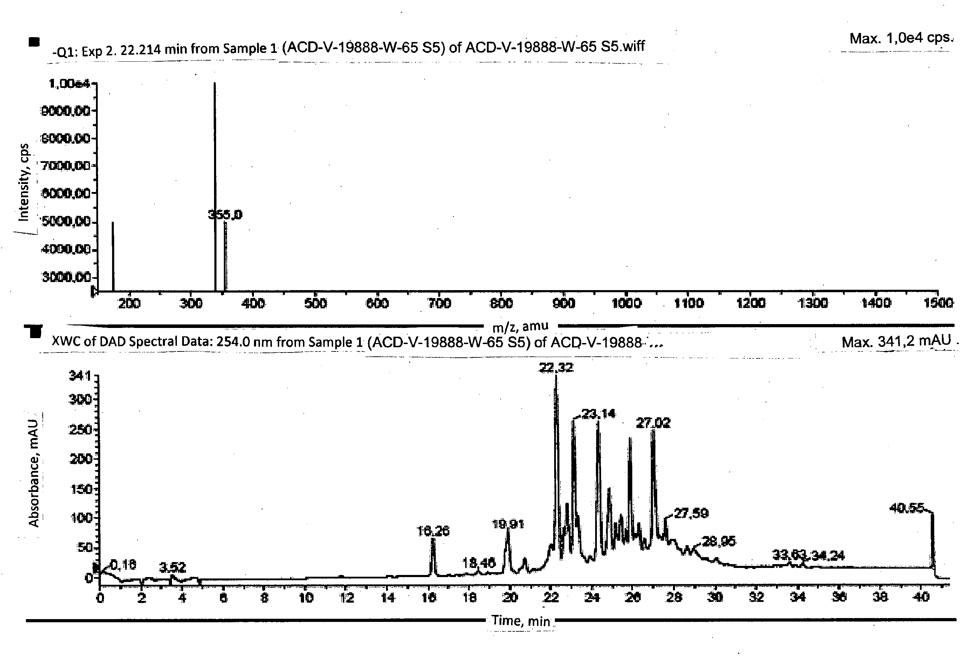

At biofilm-mid log phase (t₀) values for OD580 and CV580 were recorded and cells were incubated further until the untreated samples (control) did not show further increase in biofilm formation (stationary phase; t_{end}). Numbers for OD580 / CV580 of untreated control samples and samples treated with test compounds were compared to t₀-value, which is defined as the 100% level for planktonic growth and biofilm formation, respectively. Values for OD580 / CV580 of the untreated control sample in stationary phase (end of experiment) are defined as the "control-level" (for unrestricted growth / biofilm formation). Values for control-levels are solely dependent on t₀-values and therefore vary with each experiment; thus, they cannot be standardised. Control level values are always higher than 100%, ideally show values of about 200%, but might be even higher. The effectiveness of test compounds is given as level-% and has to be compared to the respective control-levels. Level-% values for test compounds might be close to control-level values (no effect), vary between 100% and control-level (inhibition), are close to 100 % (stagnation), or fall below 100 % (cell-lysis or biofilm detachment) (Fig. 4).

Exponentially growing cells (mid-log) are in a defined active state with respect to proliferation and metabolic activity. Cell numbers are significantly higher compared to the "classic assay", so that only highly active compounds will have an inhibitory effect on planktonic growth of *C. xerosis.* Moreover, the mode of inhibition caused by the test compound can be determined (see above).

The mid-log assay was especially designed to define the effectiveness of a test compound on biofilms that is not accessible with the classic assay. Biofilm propagation is retarded (inhibition), stopped (stagnation), or pre-formed biofilm is partially or completely dissolved (detachment). The assay can identify compounds that are active against biofilm-protected and hence persistent cells. Moreover, by combining the two read-outs of OD580 and CV580, it is possible to identify compounds selectively acting on either planktonic or biofilm cells.

### Assay conditions

1. Strain: *Corynebacterium xerosis* DSM 20743 (ATCC 373)
2. Growth conditions:
   C. xerosis is cultivated in M53-standard medium containing peptone, yeast-extract and glucose. Static incubation in flat-bottom 96-well microtiterplates (total volume 340 µl, working volume 200 µl). Incubation at 37°C for 18 - 24 h or until the untreated control entered stationary phase. Suspensions used for inoculation were adjusted to an optical density (OD580) of 0,1
3. Reference substances ("benchmarks")
   Results of test compounds were compared to Triclosan (Irgasan, 5-chloro-2-(2,4-dichlorophenoxy)-phenol)
4. Controls
   Sterile media was used as negative control; cells cultivated in absence of any test compound but in presence of equal amounts of DMSO compared to samples (see below) were regarded as positive control (unrestricted growth)
5. All compounds (test-samples and references) were dissolved in DMSO. DMSO was shown to have no negative effect on cell growth and is present in equal amounts in all samples, including untreated control samples.
6. Unless otherwise stated, test compound concentration was 12,5 µg/ml
7. Susceptibility testing was performed under conditions that allow for optimal detection of anti-biofilm compounds.

### Results:

**Planktonic growth**

| | |
|---|---|
| Primary Screening | 47 % inhibition |
| Secondary Screening | 49 % inhibition |

| IC₅₀-Determination: | |
|---|---|
| *Glycyrrhiza pallidiflora* extract | 5,0 µg/ml |
| Triclosan | 1,7 µg/ml |
| Mid-log Assay: | |
| Level-% | 62 % → cell lysis |
| Control-level % | 181 % |

**Biofilm-growth mode**

| | |
|---|---|
| Primary Screening | 0 % inhibition |
| Secondary Screening | 77 % → inhibition |

| IC₅₀-Determination: | |
|---|---|
| Licoricidin | 4,3 µg/ml |
| Triclosan | 0,6 µg/ml |
| Mid-log Assay: | |
| Level-% | 111 % → inhibition |
| Control-level % | 431 % |

### Example 7: Formulation

### Example 7.1: Deo-Creme

### A) Deo-Creme with 10 wt. % oil

| **No.** | **Ingredients** | wt. % |
|---|---|---|
| 1 | Glycerolmonostearath 60 | 4.0 |
| 2 | Cetyl alcohol | 6.0 |
| 3 | Maceration oil of *Glycyrrhiza pallidiflora* | 10.0 |
| 4 | Vaseline, white | 24.0 |
| 5 | Tagat S 2 | 7.0 |
| 6 | Propylenglycol | 10.0 |
| 5 | Water | 39.0 |
| **Preparation:** The solid ingredients were melted and mixes with the oil. This mixture was slowly added to the warm water while stirring. The mixture was homogenized slowly and packaged. | | |

### B) Deo-Creme with 25 wt. % oil

| **No.** | **Ingredients** | wt. % |
|---|---|---|
| 1 | Glycerolmonostearath 60 | 4.0 |
| 2 | Cetyl alcohol | 6.0 |
| 3 | Maceration oil of *Glycyrrhiza pallidiflora* | 25.0 |
| 4 | Vaseline, white | 16.0 |
| 5 | Tagat S 2 | 7.0 |
| 6 | Propylenglycol | 10.0 |
| 5 | Water | 32.0 |
| **Preparation:** The solid ingredients were melted and mixes with the oil. This mixture was slowly added to the warm water while stirring. The mixture was homogenized slowly and packaged.. | | |

### Example 7.2: Deo-Roll on

### A) Deo roll on (I)

| **Phase** | **No.** | **Ingredients** | wt. % |
|---|---|---|---|
| A | 1 | water | 83.15 |
| | 2 | Naviance instant Maize, Fa. Azelis Kosmetik GmbH | 3.00 |
| | 3 | Keltrol CG-SFT, Fa. Rahn AG | 0.60 |
| B | 4 | Glycerin 86,5% PH Eur pflanzl., Fa. Fauth&Co.KG | 3.00 |
| | 5 | Dermosoft 688 ECO, Fa. Dr. Straetmans | 0.10 |
| C | 6 | Dermosoft GMCY, Fa. Dr. Straetmans | 2.00 |
| | 7 | Citrofol A 1, Fa. Azelis Kosmetik GmbH | 5.00 |
| D | 8 | Oil of *Glycyrrhiza pallidiflora* (made by co-pressing with sunflower seed) | 3.00 |
| | 9 | Nivesse Nat Scent D | 0.10 |
| | 10 | Cosmaderm T-70 NON GMO, Fa. Cosphatec GmbH | 0.05 |

| **Phase A:** | | | |
|---|---|---|---|
| Ingredient No. 1 was heated to 80 °C . | | | |
| Ingredient No. 2 was added under stirring and is homogenized in vacuo for 10 min. | | | |
| Ingredient No. 3 was added under stirring and is homogenized in vacuo for 10 min.. | | | |

| **Phase B:** | | | |
|---|---|---|---|
| Ingredients No. 4+5 were mixed and subsequently added to phase A. The combined phases A and B were homogenized in vacuo for 10 min until ingredient 5 was completely dissolved. | | | |

| **Phase C:** | | | |
|---|---|---|---|
| Ingredients No. 6-8 were added. The resulting mixture was homogenized in vacuo for 10 min and cooled down to 30 ° C. | | | |

| **Phase B:** | | | |
|---|---|---|---|
| Ingredients No. 9+10 were added A/B/C and the resulting mixture was homogenized in vacuo for 10 min. | | | |
| The mixture was cooled to room temperatur. | | | |

### B) Deo Roll On (II)

| **Phase** | **No.** | **Ingredients** | wt. % |
|---|---|---|---|
| A | 1 | Steareth-2 | 4.0 |
| | 2 | Steareth-20 | 2.2 |
| | 3 | PPG-15 Stearyl Ether | 4.7 |
| B | 4 | Isopropyl Myristate | 1.0 |
| | 5 | Lipophilic Extract of *Glycyrrhiza pallidiflora* | 0.2 |
| C | 6 | Water | 87.9 |

| **Preparation:** | | | |
|---|---|---|---|
| Phase A (ingredients 1 to 3), phase B (ingredients 4 to 5) and the water were heated separetly to 65 °C. The ingredients of phase A and B were heated to 65°C and sonicated for about 15 minutes. 85 % of this mixture was slowly added to the water while stirring. The micture was homogenized slowly for 1-4 minutes while cooling to 50°C. The remaining 15% of the mixture of phase A and B were added to the batch with overhead mixing and continued mixing until homogeneous. | | | |

### Example 7.3 Deodorant-Stick

| **No.** | **Ingredients** | wt. % |
|---|---|---|
| 1 | PPG-3 Myristyl Ether | 79.0 |
| 2 | Lipophilic Extract of *Glycyrrhiza pallidiflora* | 0.3 |
| 3 | Propylene Glycol | 10.0 |
| 4 | Sodium Stearate | 8.0 |
| 5 | Water | 2.7 |
| **Preparation:** The ingredients were mixed stepwise in the given order at 80-85°C and stirred until a clear solution resulted. The mixture was cooled while stirring to approx. 75°C and packaged. | | |

## Claims

1. A composition A comprising licoricidine and at least one component selected from the group consisting of glyasperin D, glyasperin C, gancaonin I and glycyrrhisoflavone,
wherein the composition is a *Glycyrrhiza pallidiflora, Glycyrrhiza uralensis or Glycyrrhiza glabra* extract, wherein the *Glycyrrhiza* extract comprises a solvent, wherein the solvent is a plant oil and wherein the composition comprises butylhydroxyanisol.

2. The composition A according to claim 1 comprising licoricidine, glyasperin D, glyasperin C, gancaonin I and glycyrrhisoflavone.

3. The composition A according to claims 1 to 2, wherein the composition comprises licoricidine in an amount the range of from 0.05 to 10% by weight, glyasperin D in an amount in the range of from 0.04 to 8% by weight, glyasperin C in an amount in the range of from 0.01 to 4% by weight, gancaonin I in an amount in the range of from 0.025 to 5% by weight and and glycyrrhisoflavone in an amount in the range of from 0.01 to 4% by weight, based on the total weight of the composition A.

4. The composition A according to any of claims 1 to 3, wherein the *Glycyrrhiza pallidiflora, Glycyrrhiza uralensis or Glycyrrhiza glabra* extract, preferably the *Glycyrrhiza pallidiflora* or *Glycyrrhiza uralensis* extract, more preferably the *Glycyrrhiza pallidiflora* extract, is obtainable or obtained by a process comprising
(a) providing *Glycyrrhiza pallidiflora, Glycyrrhiza uralensis or Glycyrrhiza glabra,* preferably *Glycyrrhiza pallidiflora* or *Glycyrrhiza uralensis,* more preferably *Glycyrrhiza pallidiflora,* more preferably a branch and/or a root of *Glycyrrhiza pallidiflora,* and contacting the *Glycyrrhiza* material with a liquid S1 thereby forming a liquid phase L1 and a solid residue R0;
(b) separating L1 from R0;
(c) optionally drying L1 to give a residue R1;
(d) optionally dissolving the R1 in a liquid S2
wherein the liquid S1 and the liquid S2 is a plant oil, preferably a plant oil selected from the group consisting of safflower oil, sunflower oil, olive oil, rapeseed oil and mixtures thereof,
wherein the extracting in (a) is preferably carried out by a maceration
to give the *Glycyrrhiza* extract, *preferably the Glycyrrhiza pallidiflora* extract.

5. The composition A according to any of claims 1 to 3, wherein the *Glycyrrhiza pallidiflora, Glycyrrhiza uralensis or Glycyrrhiza glabra* extract, preferably the *Glycyrrhiza pallidiflora* or *Glycyrrhiza uralensis* extract, more preferably the *Glycyrrhiza pallidiflora* extract, is obtainable or obtained by a process comprising
(a1) providing *Glycyrrhiza pallidiflora, Glycyrrhiza uralensis or Glycyrrhiza glabra,* preferably *Glycyrrhiza pallidiflora* or *Glycyrrhiza uralensis,* more preferably *Glycyrrhiza pallidiflora,* more preferably a branch and/or a root of *Glycyrrhiza pallidiflora,* and contacting the *Glycyrrhiza* material with oil seeds, preferably sunflower seeds;
(a2) subjecting the mixture according to (i) to co-pressing to give a liquid phase L1 and a solid residue R0,
(a3) separating L1 from R0
to give the *Glycyrrhiza* extract, *preferably the Glycyrrhiza pallidiflora* extract.

6. A method for the preparation of a *Glycyrrhiza pallidiflora, Glycyrrhiza uralensis or Glycyrrhiza glabra* extract,
comprising
(a) providing *Glycyrrhiza pallidiflora, Glycyrrhiza uralensis or Glycyrrhiza glabra,* preferably *Glycyrrhiza pallidiflora,* more preferably a branch and/or a root of *Glycyrrhiza pallidiflora,* and contacting the *Glycyrrhiza* material with a liquid S1 thereby forming a liquid phase L1 and a solid residue R0;
(b) separating L1 from R0;
(c) optionally drying L1 to give a residue R1;
(d) optionally dissolving the R1 in a liquid S2
wherein the liquid S1 and S2 is a plant oil, preferably a plant oil selected from the group consisting of safflower oil, sunflower oil, olive oil, rapeseed oil and mixtures thereof;
preferably wherein the extracting in (a) is carried out by a maceration.
to give the *Glycyrrhiza* extract, *preferably the Glycyrrhiza pallidiflora* extract.

7. A method for the preparation of a *Glycyrrhiza pallidiflora, Glycyrrhiza uralensis or Glycyrrhiza glabra* extract, preferably a *Glycyrrhiza pallidiflora* or *Glycyrrhiza uralensis* extract, more preferably a *Glycyrrhiza pallidiflora* extract,
comprising
(a1) providing *Glycyrrhiza pallidiflora, Glycyrrhiza uralensis or Glycyrrhiza glabra,* preferably *Glycyrrhiza pallidiflora* or *Glycyrrhiza uralensis, more* preferably *Glycyrrhiza pallidiflora,* more preferably a branch and/or a root of *Glycyrrhiza pallidiflora,* and contacting the *Glycyrrhiza* material with oil seeds, preferably sunflower seeds;
(a2) subjecting the mixture according to (i) to co-pressing to give a liquid phase L1 and a solid residue R0,
(a3) separating L1 from R0
to give the *Glycyrrhiza* extract, *preferably the Glycyrrhiza pallidiflora* extract.

8. A *Glycyrrhiza pallidiflora, Glycyrrhiza uralensis or Glycyrrhiza glabra* extract, preferably a *Glycyrrhiza pallidiflora* or *Glycyrrhiza uralensis* extract, more preferably a *Glycyrrhiza pallidiflora* extract, obtainable or obtained by the method according to claims 6 or 7.

9. A pharmaceutical or cosmetic composition comprising a composition A according to any one of claims 1 to 5 or a *Glycyrrhiza* extract according to claim 8 and one or more cosmetically and/or pharmaceutically acceptable carriers and/or excipients, preferably wherein the composition is a cream, an ointment, a gel or an emulsion, more preferably wherein the composition A comprises at least one compound selected from the group consisting of glyceryl monostearate, cetyl alcohol, PEG and water.

10. A pharmaceutical or cosmetic composition comprising a composition A according to any one of claims 1 to 5 or a *Glycyrrhiza* extract according to claim 9 and one or more cosmetically and/or pharmaceutically acceptable carriers and/or excipients for use in treating or preventing dental caries.

11. A method for the preparation of pharmaceutical or cosmetic composition according to claim 9 or 10, comprising
mixing a composition according to any one of claims 1 to 5 or a *Glycyrrhiza* extract according to claim 8 with one or more cosmetically and/or pharmaceutically acceptable carriers and/or excipients.

12. The cosmetic use of a composition according to any one of claims 8 to 9 for body or oral care.

13. A pharmaceutical composition according to any one of claims 8 to 9 for use as an antibacterial composition in body or oral care.

## Patentansprüche

1. Zusammensetzung A, umfassend Licoricidin und mindestens eine Komponente ausgewählt aus der Gruppe bestehend aus Glyasperin D, Glyasperin C, Gancaonin I und Glycyrrhisoflavon,
wobei die Zusammensetzung ein Extrakt aus *Glycyrrhiza pallidiflora, Glycyrrhiza uralensis* oder *Glycyrrhiza glabra* ist, wobei der *Glycyrrhiza*-Extrakt ein Lösungsmittel umfasst, wobei das Lösungsmittel ein Pflanzenöl ist und wobei die Zusammensetzung Butylhydroxyanisol umfasst.

2. Zusammensetzung A nach Anspruch 1, umfassend Licoricidin, Glyasperin D, Glyasperin C, Gancaonin I und Glycyrrhisoflavon.

3. Zusammensetzung A nach den Ansprüchen 1 bis 2, wobei die Zusammensetzung Licoricidin in einer Menge im Bereich von 0,05 bis 10 Gew.-%, Glyasperin D in einer Menge im Bereich von 0,04 bis 8 Gew.-%, Glyasperin C in einer Menge im Bereich von 0,01 bis 4 Gew.-%, Gancaonin I in einer Menge im Bereich von 0,025 bis 5 Gew.-% und Glycyrrhisoflavon in einer Menge im Bereich von 0,01 bis 4 Gew.-%, bezogen auf das Gesamtgewicht der Zusammensetzung A, enthält.

4. Zusammensetzung A nach einem der Ansprüche 1 bis 3, wobei der Extrakt aus *Glycyrrhiza pallidiflora, Glycyrrhiza uralensis* oder *Glycyrrhiza glabra,* vorzugsweise der Extrakt aus *Glycyrrhiza pallidiflora* oder *Glycyrrhiza uralensis,* stärker bevorzugt der Extrakt aus *Glycyrrhiza pallidiflora,* durch ein Verfahren erhältlich ist oder erhalten werden kann, umfassend
(a) Bereitstellen von *Glycyrrhiza pallidiflora, Glycyrrhiza uralensis* oder *Glycyrrhiza glabra,* vorzugsweise *Glycyrrhiza pallidiflora* oder *Glycyrrhiza uralensis,* stärker bevorzugt *Glycyrrhiza pallidiflora,* stärker bevorzugt einem Zweig und/oder einer Wurzel von *Glycyrrhiza pallidiflora* und Inkontaktbringen des *Glycyrrhiza-*Materials mit einer Flüssigkeit S1, wodurch eine flüssige Phase L1 und ein fester Rückstand R0 gebildet wird;
(b) Trennen von L1 von R0;
(c) gegebenenfalls Trocknen von L1, um einen Rückstand R1 zu erhalten;
(d) gegebenenfalls Lösen von R1 in einer Flüssigkeit S2,
wobei die Flüssigkeit S1 und die Flüssigkeit S2 ein Pflanzenöl ist, vorzugsweise ein Pflanzenöl, ausgewählt aus der Gruppe bestehend aus Safloröl, Sonnenblumenöl, Olivenöl, Rapsöl und Gemischen davon,
wobei das Extrahieren in (a) vorzugsweise durch eine Mazeration durchgeführt wird, so dass der Extrakt aus Glycyrrhiza, vorzugsweise der Extrakt aus *Glycyrrhiza pallidiflora,* erhalten wird.

5. Zusammensetzung A nach einem der Ansprüche 1 bis 3, wobei der Extrakt aus *Glycyrrhiza pallidiflora, Glycyrrhiza uralensis* oder *Glycyrrhiza glabra,* vorzugsweise der Extrakt aus *Glycyrrhiza pallidiflora* oder *Glycyrrhiza uralensis,* stärker bevorzugt der Extrakt aus *Glycyrrhiza pallidiflora,* durch ein Verfahren erhältlich ist oder erhalten werden kann, umfassend
(a1) Bereitstellen von *Glycyrrhiza pallidiflora, Glycyrrhiza uralensis* oder *Glycyrrhiza glabra,* vorzugsweise *Glycyrrhiza pallidiflora* oder *Glycyrrhiza uralensis,* stärker bevorzugt *Glycyrrhiza pallidiflora,* stärker bevorzugt einem Zweig und/oder einer Wurzel von *Glycyrrhiza pallidiflora* und Inkontaktbringen des *Glycyrrhiza-*Materials mit Ölsamen, vorzugsweise Sonnenblumensamen;
(a2) gemeinsames Pressen des Gemischs gemäß (i), so dass eine flüssige Phase L1 und ein fester Rückstand R0 erhalten wird,
(a3) Trennen von L1 von R0,
so dass der Extrakt aus *Glycyrrhiza,* vorzugsweise der Extrakt aus *Glycyrrhhiza pallidiflora,* erhalten wird.

6. Verfahren zur Herstellung eines Extraktes aus *Glycyrrhiza pallidiflora, Glycyrrhiza uralensis* oder *Glycyrrhiza glabra,*
umfassend
(a) Bereitstellen von *Glycyrrhiza pallidiflora, Glycyrrhiza uralensis* oder *Glycyrrhiza glabra,* vorzugsweise *Glycyrrhiza pallidiflora,* stärker bevorzugt einem Zweig und/oder einer Wurzel von *Glycyrrhiza pallidiflora* und Inkontaktbringen des Glycyrrhiza-Materials mit einer Flüssigkeit S1, wodurch eine flüssige Phase L1 und ein fester Rückstand R0 gebildet wird;
(b) Trennen von L1 von R0;
(c) gegebenenfalls Trocknen von L1, um einen Rückstand R1 zu erhalten;
(d) gegebenenfalls Lösen von R1 in einer Flüssigkeit S2,
wobei die Flüssigkeit S1 und S2 ein Pflanzenöl ist, vorzugsweise ein Pflanzenöl, ausgewählt aus der Gruppe bestehend aus Safloröl, Sonnenblumenöl, Olivenöl, Rapsöl und Gemischen davon,
wobei das Extrahieren in (a) vorzugsweise durch eine Mazeration durchgeführt wird,
so dass der Extrakt aus *Glycyrrhiza,* vorzugsweise der Extrakt aus *Glycyrrhiza pallidiflora,* erhalten wird.

7. Verfahren zur Herstellung eines Extraktes aus *Glycyrrhiza pallidiflora, Glycyrrhiza uralensis* oder *Glycyrrhiza glabra,* vorzugsweise eines Extraktes aus *Glycyrrhiza pallidiflora* oder *Glycyrrhiza uralensis,* stärker bevorzugt eines Extraktes aus *Glycyrrhiza pallidiflora,*
umfassend
(a1) Bereitstellen von *Glycyrrhiza pallidiflora, Glycyrrhiza uralensis* oder *Glycyrrhiza glabra,* vorzugsweise *Glycyrrhiza pallidiflora* oder *Glycyrrhiza uralensis,* stärker bevorzugt *Glycyrrhiza pallidiflora,* stärker bevorzugt einem Zweig und/oder einer Wurzel von *Glycyrrhiza pallidiflora* und Inkontaktbringen des *Glycyrrhiza-*Materials mit Ölsamen, vorzugsweise Sonnenblumensamen;
(a2) gemeinsames Pressen des Gemischs gemäß (i), wobei eine flüssige Phase L1 und ein fester Rückstand R0 erhalten wird,
(a3) Trennen von L1 von R0, so dass der Extrakt aus *Glycyrrhiza,* vorzugsweise der Extrakt aus *Glycyrrhhiza pallidiflora,* erhalten wird.

8. Extrakt aus *Glycyrrhiza pallidiflora, Glycyrrhiza uralensis* oder *Glycyrrhiza glabra,* vorzugsweise ein Extrakt aus *Glycyrrhiza pallidiflora* oder *Glycyrrhiza uralensis,* stärker bevorzugt ein Extrakt aus *Glycyrrhiza pallidiflora,* erhältlich oder erhalten durch ein Verfahren nach den Ansprüchen 6 oder 7.

9. Pharmazeutische oder kosmetische Zusammensetzung, umfassend eine Zusammensetzung A nach einem der Ansprüche 1 bis 5 oder einen *Glycyrrhiza*-Extrakt nach Anspruch 8 und einen oder mehrere kosmetisch und/oder pharmazeutisch akzeptable Träger und/oder Exzipienten, wobei die Zusammensetzung vorzugsweise eine Creme, eine Salbe, ein Gel oder eine Emulsion ist, wobei die Zusammensetzung A stärker bevorzugt mindestens eine Verbindung umfasst, die ausgewählt ist aus der Gruppe bestehend aus Glycerylmonostearat, Cetylalkohol, PEG und Wasser

10. Pharmazeutische oder kosmetische Zusammensetzung, umfassend eine Zusammensetzung A nach einem der Ansprüche 1 bis 5 oder einen *Glycyrrhiza*-Extrakt nach Anspruch 9 und einen oder mehrere kosmetisch und/oder pharmazeutisch akzeptable Träger und/oder Exzipienten zur Verwendung bei der Behandlung oder Vorbeugung von Zahnkaries.

11. Verfahren zur Herstellung einer pharmazeutischen oder kosmetischen Zusammensetzung nach Anspruch 9 oder 10, umfassend
Mischen einer Zusammensetzung nach einem der Ansprüche 1 bis 5 oder eines *Glycyrrhiza*-Extrakts nach Anspruch 8 mit einem oder mehreren kosmetisch und/oder pharmazeutisch akzeptablen Trägern und/oder Exzipienten.

12. Kosmetische Verwendung einer Zusammensetzung nach einem der Ansprüche 8 bis 9 zur Körper- oder Mundpflege.

13. Kosmetische Verwendung einer Zusammensetzung nach einem der Ansprüche 8 bis 9 zur Verwendung als antibakterielle Zusammensetzung bei der Körper- oder Mundpflege.

## Revendications

1. Composition A comprenant de la licoricidine et au moins un composant choisi dans le groupe constitué par la glyaspérine D, la glyaspérine C, la gancaonine I et la glycyrrhisoflavone,
où la composition est un extrait de *Glycyrrhiza pallidiflora,* de *Glycyrrhiza uralensis* ou de *Glycyrrhiza glabra,* où l'extrait de *Glycyrrhiza* comprend un solvant, où le solvant est une huile végétale et où la composition comprend du butylhydroxyanisol.

2. Composition A selon la revendication 1 comprenant de la licoricidine, de la glyaspérine D, de la glyaspérine C, de la gancaonine I et de la glycyrrhisoflavone.

3. Composition A selon les revendications 1 à 2, où la composition comprend de la licoricidine dans une proportion comprise dans l'intervalle allant de 0,05 à 10 % en masse, de la glyaspérine D dans une proportion comprise dans l'intervalle allant de 0,04 à 8 % en masse, de la glyaspérine C dans une proportion comprise dans l'intervalle allant de 0,01 à 4 % en masse, de la gancaonine I dans une proportion comprise dans l'intervalle allant de 0,025 à 5 % en masse et de la glycyrrhisoflavone dans une proportion comprise dans l'intervalle allant de 0,01 à 4 % en masse, par rapport à la masse totale de la composition A.

4. Composition A selon l'une quelconque des revendications 1 à 3, où l'extrait de *Glycyrrhiza pallidiflora,* de *Glycyrrhiza uralensis* ou de *Glycyrrhiza glabra,* préférentiellement l'extrait de *Glycyrrhiza pallidiflora* ou de *Glycyrrhiza uralensis,* plus préférentiellement l'extrait de *Glycyrrhiza pallidiflora,* peut être obtenu ou est obtenu par un procédé comprenant les étapes consistant à
(a) se munir de *Glycyrrhiza pallidiflora,* de *Glycyrrhiza uralensis* ou de *Glycyrrhiza glabra,* préférentiellement de *Glycyrrhiza pallidiflora* ou de *Glycyrrhiza uralensis,* plus préférentiellement de *Glycyrrhiza pallidiflora,* plus préférentiellement d'une branche et/ou d'une racine de *Glycyrrhiza pallidiflora,* et mettre en contact le matériel de *Glycyrrhiza* avec un liquide S1 pour former une phase liquide L1 et un résidu solide R0 ;
(b) séparer L1 de R0 ;
(c) éventuellement sécher L1 pour obtenir un résidu R1 ;
(d) éventuellement dissoudre le R1 dans un liquide S2
où le liquide S1 et le liquide S2 sont une huile végétale, préférentiellement une huile végétale choisie dans le groupe constitué par l'huile de carthame, l'huile de tournesol, l'huile d'olive, l'huile de colza et leurs mélanges,
où l'extraction de (a) est préférentiellement mise en oeuvre par une macération pour obtenir l'extrait de *Glycyrrhiza,* préférentiellement l'extrait de *Glycyrrhiza pallidiflora.*

5. Composition A selon l'une quelconque des revendications 1 à 3, où l'extrait de *Glycyrrhiza pallidiflora,* de *Glycyrrhiza uralensis* ou de *Glycyrrhiza glabra,* préférentiellement l'extrait de *Glycyrrhiza pallidiflora* ou de *Glycyrrhiza uralensis,* plus préférentiellement l'extrait de *Glycyrrhiza pallidiflora,* peut être obtenu ou est obtenu par un procédé comprenant les étapes consistant à
(a1) se munir de *Glycyrrhiza pallidiflora,* de *Glycyrrhiza uralensis* ou de *Glycyrrhiza glabra,* préférentiellement de *Glycyrrhiza pallidiflora* ou de *Glycyrrhiza uralensis,* plus préférentiellement de *Glycyrrhiza pallidiflora,* plus préférentiellement d'une branche et/ou d'une racine de *Glycyrrhiza pallidiflora,* et mettre en contact le matériel de *Glycyrrhiza* avec des graines oléagineuses, préférentiellement des graines de tournesol ;
(a2) soumettre le mélange selon (i) à une co-pression pour obtenir une phase liquide L1 et un résidu solide R0,
(a3) séparer L1 de R0
pour obtenir l'extrait de *Glycyrrhiza,* préférentiellement l'extrait de *Glycyrrhiza pallidiflora.*

6. Procédé de préparation d'un extrait de *Glycyrrhiza pallidiflora,* de *Glycyrrhiza uralensis* ou de *Glycyrrhiza glabra,*
comprenant les étapes consistant à
(a) se munir de *Glycyrrhiza pallidiflora,* de *Glycyrrhiza uralensis* ou de *Glycyrrhiza glabra,* préférentiellement de *Glycyrrhiza pallidiflora,* plus préférentiellement d'une branche et/ou d'une racine de *Glycyrrhiza pallidiflora,* et mettre en contact le matériel de *Glycyrrhiza* avec un liquide S1 pour former une phase liquide L1 et un résidu solide R0 ;
(b) séparer L1 de R0 ;
(c) éventuellement sécher L1 pour obtenir un résidu R1 ;
(d) éventuellement dissoudre le R1 dans un liquide S2 où les liquides S1 et S2 sont une huile végétale, préférentiellement une huile végétale choisie dans le groupe constitué par l'huile de carthame, l'huile de tournesol, l'huile d'olive, l'huile de colza et leurs mélanges ;
préférentiellement où l'extraction de (a) est mise en oeuvre par macération,
pour obtenir l'extrait de *Glycyrrhiza,* préférentiellement l'extrait de *Glycyrrhiza pallidiflora.*

7. Procédé de préparation d'un extrait de *Glycyrrhiza pallidiflora,* de *Glycyrrhiza uralensis* ou de *Glycyrrhiza glabra,* préférentiellement d'un extrait de *Glycyrrhiza pallidiflora* ou de *Glycyrrhiza uralensis,* plus préférentiellement d'un extrait de *Glycyrrhiza pallidiflora,*
comprenant les étapes consistant à
(a1) se munir de *Glycyrrhiza pallidiflora,* de *Glycyrrhiza uralensis* ou de *Glycyrrhiza glabra,* préférentiellement de *Glycyrrhiza pallidiflora* ou de *Glycyrrhiza uralensis,* plus préférentiellement de *Glycyrrhiza pallidiflora,* plus préférentiellement d'une branche et/ou d'une racine de *Glycyrrhiza pallidiflora,* et mettre en contact le matériel de *Glycyrrhiza* avec des graines oléagineuses, préférentiellement des graines de tournesol ;
(a2) soumettre le mélange selon (i) à une co-pression pour obtenir une phase liquide L1 et un résidu solide R0,
(a3) séparer L1 de R0
pour obtenir l'extrait de *Glycyrrhiza,* préférentiellement l'extrait de *Glycyrrhiza pallidiflora.*

8. Extrait de *Glycyrrhiza pallidiflora,* de *Glycyrrhiza uralensis* ou de *Glycyrrhiza glabra,* préférentiellement extrait de *Glycyrrhiza pallidiflora* ou de *Glycyrrhiza uralensis,* plus préférentiellement extrait de *Glycyrrhiza pallidiflora,* qui peut être obtenu ou est obtenu par le procédé selon les revendications 6 ou 7.

9. Composition pharmaceutique ou cosmétique comprenant une composition A selon l'une quelconque des revendications 1 à 5 ou extrait de *Glycyrrhiza* selon la revendication 8 et un ou plusieurs véhicules et/ou excipients cosmétiquement et/ou pharmaceutiquement acceptables, préférentiellement où la composition est une crème, un onguent, un gel ou une émulsion, plus préférentiellement où la composition A comprend au moins un composé choisi dans le groupe constitué par le monostéarate de glycéryle, le cétol, le PEG et de l'eau.

10. Composition pharmaceutique ou cosmétique comprenant une composition A selon l'une quelconque des revendications 1 à 5 ou extrait de *Glycyrrhiza* selon la revendication 9 et un ou plusieurs véhicules et/ou excipients cosmétiquement et/ou pharmaceutiquement acceptables pour utilisation dans le traitement prophylactique ou thérapeutique des caries dentaires.

11. Procédé de préparation d'une composition pharmaceutique ou cosmétique selon la revendication 9 ou 10, comprenant les étapes consistant à mélanger une composition selon l'une quelconque des revendications 1 à 5 ou un extrait de *Glycyrrhiza* selon la revendication 8 avec un ou plusieurs véhicules et/ou excipients cosmétiquement et/ou pharmaceutiquement acceptables.

12. Utilisation cosmétique d'une composition selon l'une quelconque des revendications 8 à 9 pour soins corporels ou oraux.

13. Composition pharmaceutique selon l'une quelconque des revendications 8 à 9 pour utilisation en tant que composition antibactérienne dans des soins corporels ou oraux.
